(19) **Europäisches Patentamt / European Patent Office / Office européen des brevets**

(11) **EP 3 156 045 A1**

(12) # EUROPEAN PATENT APPLICATION
published in accordance with Art. 153(4) EPC

(43) Date of publication:
**19.04.2017 Bulletin 2017/16**

(21) Application number: **15810603.9**

(22) Date of filing: **05.06.2015**

(51) Int Cl.:
**A61K 9/107** *(2006.01)*    **A61K 47/10** *(2017.01)*

(86) International application number:
**PCT/CN2015/080882**

(87) International publication number:
**WO 2015/192720 (23.12.2015 Gazette 2015/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **16.06.2014   CN 201410273576**

(71) Applicants:
• **Shenyang Pharmaceutical University**
  **Shenyang, Liaoning 110016 (CN)**
• **Deliwei (Beijing) Biological Technology Co., Ltd**
  **Beijing 100044 (CN)**

(72) Inventors:
• **DENG, Yihui**
  **Shenyang**
  **Liaoning 110016 (CN)**

• **CHENG, Xiaobo**
  **Beijing 100044 (CN)**
• **WANG, Yu**
  **Shenyang**
  **Liaoning 110016 (CN)**
• **DENG, Jilin**
  **Beijing 100044 (CN)**
• **WU, Ying**
  **Beijing 100044 (CN)**
• **WU, Baozhen**
  **Beijing 100044 (CN)**

(74) Representative: **Gill, Siân Victoria et al**
  **Venner Shipley LLP**
  **200 Aldersgate**
  **London EC1A 4HD (GB)**

(54)  **APPLICATION OF LARGE-DOSE GLYCERINUM IN FREEZE-THAWING TOLERABLE LIPID EMULSION**

(57)    The invention relates to fat emulsions, particularly to a use of high-concentrationglycerol in freeze-thaw resistant emulsions and a free-thaw resistant emulsion thereof. The said high-concentrationglycerol is the glycerol that is greater than or equal to 3 w/v % in the emulsion composition. The maximum percentage of the glycerol in the emulsion is 50 w/v %. When the percentage of the oil in the emulsion is 2%-30 w/v %, the glycerol is more than or equal to 1/3 of the oil in the emulsion. The invention comprises adrug-contained emulsion through including drugs. Compared with prior arts, the invention provides a freeze-thaw resistant emulsion which tolerates the low-temperature freeze-thaw experiments, avoiding the pharmaceutical stability issues due to the temperature changes during the emulsion transport, storage and utilization, ensuring medicine quality, meanwhile it drastically reduces the requirements of the transport and storage conditions as well as the medicine costs.

## Description

### Field of the Invention

The present invention relates to the technical field of fat emulsions, in particular, to a method of using high-concentration glycerol in freeze-thaw fat emulsions and a freeze-thaw fat emulsion thereof.

### Background of the Invention

In 1961, Swedish Prof. Wretlind first produced fat emulsions for intravenous injection from soya bean oil and egg yolk phosphatidylcholine (EPC), marking the application of the fat emulsions in the parenteral nutrition. As the earliest nutrients intravenous injection, the fat emulsions have been in the clinical use for nearly half a century and provided energy and essential fatty acids for the patients who cannot eat or are severely nutrition-deficient. Currently some fat emulsion injections have been in sale in China, such as long-chain fat emulsion injection, medium-chain/long-chain fat emulsion injection and structural fat emulsion injection, etc. with more than 100 approved numbers and over 20 approved manufacturing enterprises. In addition, the fat emulsions can be used as vehicles to increase the drug bioavailability/ or targeting, reduce toxicity and irritation, and they have been utilized widely in many aspects including anti-tumor drugs, anti-microbial drugs, and cardiovascular drugs. Until now, there are many emulsions in domestic and oversea markets, e.g. diazepam emulsion for injection, propofol emulsion for injection, perfluorocarbon injection, etomidate fat emulsion injection, prostaglandin $E_1$ emulsion injection, compound fat-soluble vitamin emulsion injection and dexamethasone palmitate emulsion injection, etc. In fact, all oil substances can be made into emulsions, e.g. Chinese Invention Patent (ZL 200510046592.1) discloses the making of the bacteria-filtered herbal or animal or plant volatile oils or lipid emulsions through compound emulsifiers, ensure the sterilization stability of the volatile substances and increase the concentrations of non-ionic surfactants. Many studies on the drug-contained fat emulsions have been available, e.g. docetaxel emulsion, compound *Bruceajavanica* seed oil emulsion, ginsenoside C-K emulsion, zedoaryturmeric oil emulsion, cinobufotalin emulsion, garlic oil emulsion, acyclovir emulsion, zidovudinepalmitate emulsion, curcumol emulsion, itraconazole emulsion, butyl-phthalideemlusion, prostaglandin $E_1$ emulsion, nimodipine emulsion, tanshinone IIA, multiplex propofol sub-micron emulsion, ketoprofen isopropyl ester emulsion, volatile oil in Radix Bupleuri emulsion, vitamin E emulsion, vitamin $K_1$ emulsion, $CoQ_{10}$ emulsion, etc.

Interestingly the inventors find that, except few fat emulsions (Liposyn III 30 (contains 30% soybean oil,1.8% egg phosphatides and 2.5% glycerol, pH 8.4 (6.0 to 9.0))), the market-selling fat emulsions unanimously select egg phosphatidylcholine (EPC), and the majority of the concentrations of the EPC are 1.2% when taking comprehensive survey in market-selling fat emulsions. And we also find that the concentration of the glycerol in the fat emulsion is less than 2.5%, e.g. 2.2% for the 20% fat emulsion injection (Sichuan Kelun Pharmaceutical Co., Ltd), 1.67% for the 30% fat emulsion injection (Xi'an Libang Pharmaceutical Co., Ltd), medium-chain and long-chain fat emulsion injection (Germany/B.Braun Medical (Suzhou)) Company) (LM-20-2.5,10% LCT, 10% MCT, 1.2% EPC,2.5% glycerol), Structural fat emulsion (Wuxi SSPC)(STG-20-2.2, 20% STG, 1.2% EPC, 2.2% glycerol), 2.5% glycerol for fish oil fat emulsion injection (OMEGAVEN, SSPC). Searching on http://www.rxlist.com, we find the fat emulsion intravenous injection contains Liposyn II (20%) (10% cardy oil, 5% soy bean oil, 1.2% EPC and 2.5% glycerol), Liposyn II (10%) (5% cardy oil, 5% soy bean oil, 1.2% EPC and 2.5% glycerol), Liposyn III (10%)(10% soy bean oil, 1.2% EPC and 2.5 glycerol), INTRALIPID® (20%)(20% soy bean, 1.2% EPC and 2.25% glycerol), INTRALIPID® (10%) (10% soy bean, 1.2% EPC and 2.25% glycerol), CLINOLIPID (20%) (16% olive oil, 4% soybean oil, 1.2% EPC,0.03 sodium oleate and 2.25% glycerol), among which the glycerol is no more than 2.5%. The case is similar for the drug-contained fat emulsions (drug-contained emulsions), e.g. diprivan emulsion (propofol emulsion) (10% soybean oil, 1.2% EPC, 2.25% glycerol and 0.005% EDTA-2Na); Cleviprex emulsion (20% soybean oil, 1.2% EPC, 0.03% oleic acid, 2.25% glycerol and 0.005% EDTA-2Na); VITALIPID N for adults and children (only different in vitamin A and other active ingredients) (10% soybean oil, 1.2% EPC and 2.2% glycerol).

In summary, it's easy to find that, in the fat emulsion concerning products available, the phospholipids (1.2% and 1.8%) and glycerol (1.67%, 2.2%, 2.25% and 2.5%) seem an established rule, i.e. the phospholipids is mainly 1.2% (few in 1.8%), glycerol no more than 2.5%. It indicates that researchers / manufacturers have not taken insight into these products to avoid some puzzling phenomena, e.g. the 30% soybean oil fat emulsion of Xi'an Libang is 1.67% in glycerol, and meanwhile, the Liposyn III 30of 30% soybean oil is 2.5% in glycerol. More interestingly, the glycerol in all fat emulsions is no more than 2.5%, i.e. the glycerol is used only as an osmotic pressure regulator as long as to meet the clinical requirements of the emulsions. In general, the common large-volume injection in use is 1-2 in osmotic pressure, e.g. 5% glucose injection (1 osmotic pressure) and 10% glucose injection (2 osmotic pressures), and the isotonic concentration of the glycerol is around 2.5% (The glycerol of 1-2 osmotic pressures is 2.5%-5.0%), combining the osmotic pressure arising from the emulsion drop in oil phase, therefore the glycerol concentration in the fat emulsion never exceeds 2.5%, and the amount of the glycerol is not rigid.

The phospholipid is another element to be reconsidered. Theoretically, the surfaces of the emulsions are completely covered by the emulsifier molecules (phospholipids) and the emulsion will reach the most stable state. The size of the emulsion particles, oil concentration and the surface area of the heads of the phospholipids are influencing the concentration of the phospholipids (emulsifier) entirely covering the emulsion drops. Generally the head surface area of the phospholipids is about 0.3-0.5nm$^2$ (even 0.75nm$^2$Colloids and Surfaces B:Biointerfaces 37 (2004) 43-47), and the market fat emulsions are presumed in particle size as 300nm (200-300nm for most of the particle sizes), Based on the surface areas of 0.3 nm$^2$, 0.4 nm$^2$ and 0.5 nm$^2$, the concentration of the phospholipids is : for 10% emulsions, 0.938g% (0.3 nm$^2$),0.705g% (0.4 nm$^2$) and 0.56g% (0.5 nm$^2$); for 20% emulsions, .88g% (0.3 nm$^2$),1.4g% (0.4 nm$^2$) and 1.1g% (0.5 nm$^2$) ; for 30% emulsions, 2.8g % (0.3 nm$^2$), 2.1g% (0.4 nm$^2$) and 1.6g% (0.5 nm$^2$).Obviously the phospholipids are different for different head surface areas. In addition, the concentration of the phospholipids vary for different oil contents, therefore it seems unreasonable for the uniform 1.2% EPC in the marketing fat emulsions. When the concentration of the phospholipids is fixed, the phospholipids in emulsifying the drops are decreasing as the oil concentration goes down, the rest of the phospholipids will form liposomes in the water. Haumont D et al Effect of liposomal content of lipid emulsions on plasma lipid concentrations in low birth weight infants receiving parenteral nutrition. J Pediatr. 1992 Nov;121(5 Pt 1):759-63.) suggests that , due to the two times liposomal content in marketing 10% emulsions as that in the 20% emulsions(note, the phospholipids are the same in 10% and 20% emulsions, i.e. 1.2%),plasma lipid levels in preterm infants will undergo a higher alternation, it is recommended about using 20% fat emulsions.

Based on the prior arts, whatever type of(common/nutrition) the fat emulsions is, injections or drug-contained fat emulsions, they will lose their effectiveness after freezing destroys their structures as the oil floats or oil-water separates, i.e. freeze-thaw vulnerable. Therefore the market-selling product manuals warn that the product cannot be reused after freezing, and the storage temperature cannot be lower than 0 °C (usually 4 °C-8 °C). The temperatures in most areas of China are below 0°C in winter,even lower than -30°C, the market-selling emulsions in transport and storage shall be protected by "special system" called "cold chain". Although the Chinese invention patent (A freeze-thaw resistant emulsion platform, application NO.201210455782.9) discloses an emulsion tolerating the freeze-thaw experiment by combining the compound emulsifiers and antifreeze agents, but the emulsions contain not only the routine emulsifiers and phospholipids, but also the non-phospholipids emulsifiers (e.g. HS15), and anti-freeze agents. It is very complicated, and the inclusion of the non-phospholipids emulsifiers and anti-freeze agents is bound to increasing the complication of the further quality research and drug safety. Until now, there are no freeze-thaw resistant nutrition-type fat emulsions and drug-contained fat emulsion products.

In prior arts, 30% emulsions are often coupled with the emulsifiers, oleic acid /sodium oleate to stabilize the physical properties of the preparation. But inventors have found that,even adding the sodium oleate cannot resist the destruction from the freeze-thaw experiments. Although some studies have demonstrated (Lipid emulsions as a novel system to reduce the hemolytic activity of lytic, agents: mechanism of the protective effect, European Journal of Pharmaceutical Sciences 9 (2000) 285-290) that emulsions can eliminate the hemolysis caused by oleic acid (0.005% sodium oleate will cause 100% hemolysis.), however, if the surfaces of the emulsion drops are not covered by the phospholipids and glycerol completely, the oleic acid/sodium oleate contained fat emulsions still pose a hemolytic risk. It's stressed that the oil concentration is higher, then the emulsion is more easily destroyed, e.g. the damages of 10%, 20% and 30% fat emulsions are increasing as the concentration grows.

## Description of the Invention

For the insufficiency and setbacks of the prior arts, i.e. the fat emulsions available cannot tolerate the freeze-thaw treatment, and the compatibility of the formulations changes and the emulsion is easily to aggregate. The invention is to provide a method of making the fat emulsions freeze-thaw resistant and a freeze-thaw resistant fat emulsion.

To realize the objective of the invention, the inventors provide the technical schemes as follows:

A use of high-concentration glycerol in freeze-thaw resistant fat emulsions, in which, the concentration of the high-concentration glycerol in the emulsions is greater than or equal to 3 w/v %.

Till now, the glycerol is only used to regulate the osmotic pressure in the emulsions, neglecting its other values, the inventors have not found any report on the glycerol in solving the common issues of the fat emulsions, which must be faced and overcome during their clinical use, including freeze-thaw issue, combinations, and particle aggregates. The inventors accidentally found, apart from the regulating the osmotic pressure, the glycerol higher than the routine concentration will produce unexpected effects to solve the problems. If the glycerol of the market-selling fat emulsions and drug-contained emulsions reach 3w/v% or higher, it will become a freeze-thaw resistant fat emulsion (note: the fat emulsions purchased from the hospitals cannot resist the freeze-thaw destruction), and meanwhile, the concentration of the glycerol is not limitless, when the glycerol reaches 40% (w/v%), the uniformity of the particle distribution in the system is decreasing.

The invention can be used to produce drug-contained or drug-free freeze-thaw resistant fat emulsions for intravenous injection or oral administration.

As a preferred scheme, the invention provides a use of high-concentration glycerol in freeze-thaw resistant fat emulsions, in which, the concentration of the high-concentration glycerol in the emulsions is greater than or equal to 3 w/v %.(Mass Volume Fraction) the maximum concentration of the high-concentration glycerol in the emulsions is 50 w/v%, preferably 5-40%, more preferably 7.5-30%, most preferably 7.5%-15%.

As a preferred scheme, the use of high-concentration glycerol in freeze-thaw resistant fat emulsions in the invention, in which, the freeze-thaw resistant fat emulsions pay more attention to the ratio of the oil and glycerol based on current classic emulsion formulations, in which, the glycerol is greater than or equal to the 1/3 amount of the oil in the freeze-thaw resistant fat emulsions where the concentration of the oil is 2%-10 w/v %.

As a preferred scheme, the use of high-concentration glycerol in freeze-thaw resistant fat emulsions in the invention, in which, the freeze-thaw resistant fat emulsions pay more attention to the ratio of the oil and glycerol based on current classic emulsion formulations, in which, the glycerol is greater than or equal to the 1/3 amount of the oil in the freeze-thaw resistant fat emulsions where the concentration of the oil is 10%-30 w/v %.

The invention also provides a freeze-thaw fat emulsion, comprising oil, glycerol, phospholipids and water, in which the concentration of the glycerol in the emulsion is greater than or equal to 3 w/v %.

As a preferred scheme, the freeze-thaw fat emulsion of the said invention, in which, the maximum concentration of the glycerol in the emulsion is 50 w/v %, preferably5-40%, more preferably 7.5%-30%, most preferably 7.5%-15%.

As a preferred scheme, the freeze-thaw fat emulsion of the said invention, in which, the concentration of the glycerol is greater than or equal to 1/3 amount of the oil in the emulsion where the concentration of the oil in the emulsion is 2 w/v %-10 w/v %.

As a preferred scheme, the freeze-thaw fat emulsion of the said invention, in which, the concentration of the glycerol is greater than or equal to 1/3 amount of the oil in the emulsion where the concentration of the oil in the emulsion is 10 w/v %-30 w/v %.

As a preferred scheme, the freeze-thaw fat emulsion of the said invention, in which, the freeze-thaw resistant fat emulsion comprises pH regulator, and the concentration of the pH regulator enables the emulsion to be pH 4.5-10.1

As a preferred scheme, the freeze-thaw fat emulsion of the said invention, in which, the freeze-thaw resistant fat emulsion comprises anti-oxidants.

In the said invention, the oil is one or a mixture of plant oil, animal oil, volatile oil and synthetic oil. The plant oil maybe soybean oil, olive oil, sunflower seed oil, cardy oil, tea seed oil , maize oil, cotton seed oil, peanut oil, refined almond oil, sesame oil, *Bruceajavanica*seed oil , coix seed oil , perilla oil , evening primrose seed oil , seabuckthorn seed oil; Thevolatile oil may be zedoaryturmeric oil, elemene, garlic oil, angelica oil, ganoderma spore oil, celery oil; the animal oil may be fish oil, seal oil, shrimp oil; the synthetic oil maybe medium-chain triglyceride (MCT) and structural oil (STG).

In the said invention, the phospholipids include natural phospholipids, semi-synthetic phospholipids and whole-synthetic phospholipids. And the natural phospholipidsmay be soya lecithin (SPC), Egg lecithin (EPC), and sphingomyelin (SM), preferably the EPC; the semi-synthetic phospholipids may be hydrogenated soybean phosphatidylcholine (HSPC) and hydrogenated egg phosphatidylcholine (HEPC); the whole-synthetic phospholipids may be DOPC, DOPG, DPPC, DPPG, DSPC, DSPG, DMPC, DMPG, etc.

In the said invention, the pH regulator may be inorganic acids (hydrochloric acid, phosphoric acid and carbonic acid), organic acids (acetic acid, tartaric acid, citric acid, malic acid, oxalic acid, lactic acid, fumaric acid, maleic acid, succinic acid, aspartic acid, glutamic acid, glycine, alanine, leucine, isoleucine, valine, cystine, cysteine, methionine, threonine, serine, phenylalanine, tyrosine, tryptophane, proline,methionine and hydroxyproline); inorganic base (sodium hydroxide), organic base (asparamide, glutamine, lysine, arginine, histidine and betaine) and the sodium salts thereof (disodium hydrogen phosphate, sodium dihydrogen phosphate, sodium phosphate, sodium carbonate, sodium bicarbonate, sodium acetate, sodium tartrate, citric sodium, sodium malate, sodium oxalate, sodium lactate, sodium fumarate, sodium maleate and sodium succinate).

In the said invention, the antioxidant may be sulfite, hydrosulfite, metasulfite, dithiocarbamate, ascorbic acid, ascorbylpalmitate, cystine,tocopherol, vitamin E, propyl gallate, butylatedhydroxyarisol, butylatedhydroxytoluene, EDTA-2Na and EDTA CaNa.

In the said invention, the water may be purified water, distilled water, injection water, sterile injection water, the quantity of which is the rest of the emulsion apart from the oil, glycerol, phospholipids, pH regulator and antioxidant.

The freeze-thaw resistant emulsions of the said invention may be drug-contained emulsions, in which the drug may be :dexamethasone palmitate, CoQ10, propofol, anisole, asarone, elemene, curcumin, tanshinone IIA, prostaglandin E1, limaprost, ketoprofen isopropyl ester, malotilate, vitamin K1, cucurbitacin E, and the concentration of the glycerol is greater than or equal to 4.5 w/v %, the maximum glycerol is the 40% of the emulsion, preferably 5%-40%, more preferably 5%-30%, most preferably 5%-20%.

Compared with prior arts, the said invention has the beneficial effects as follows:

1. The said invention provides a freeze-thaw resistant fat emulsion tolerating low-temperature freeze-thaw, avoiding the issues arising from emulsions transport, storage and the drug stability problem during utilization caused by temperature changes, which ensures the drug quality as well as lowers the requirements of the transport and storage conditions, and finally reduces the drug costs.

2. The said invention provides a freeze-thaw resistant fat emulsion which increases the stability of shaking.

3. The said invention provides a freeze-thaw resistant fat emulsion which increases the concentration of the glycerol and reduces the pH shift after the emulsion sterilization, enhancing the pH management; the said invention also resists the damages from metal ions for the industrial production and optimizes the compatibility stability of the metal ions in the emulsion.

4. The said invention provides a freeze-thaw resistant fat emulsion which adopts irregular concentrations of the glycerol, i.e. increasing to greater than or equal to 3%, improving the chemical stability of the drug in the drug-contained emulsions.

5. The said invention provides a freeze-thaw resistant fat emulsion to decrease PFAT5, especially the compatibility with the plastic containers, improving the product quality and the drug safety, especially elevating the drug safety for the newborn / senior people.(The blood vessels of the senior people are less elastic, and the capillary vessels are narrower/ even partly blocked , vulnerable to big emulsion particles (PFAT5)).

6. The said invention provides a freeze-thaw resistant fat emulsion which is altered simply on the glycerol concentration based on the emulsion formula available without adding other materials. Therefore it's easily to be operated and ready for the industrialized production.

## Detailed Description of the Preferred Embodiments

The following embodiments are to give a detailed description of the said invention. It is understood that the following embodiments shall not be for the use in limiting the invention, any form of the variations and /or modification concerning the invention shall fall within the scope of the invention.

In the said invention, any part or percentage shall refer to the weight unit, and all equipment and materials can be purchased in the market or are commonly used in the trade, unless otherwise specified. The methods employed in the experiments are the general techniques of the art, unless otherwise specified.

Note:

In the freeze-thaw experiment, the inventor evaluates the stability by freeze-thaw stability constant $K_F$, the formula is as follows:

$$K_F = \frac{\sqrt{\frac{1}{n-1} \times \sum\limits_{i=1}^{n} (D_i - \overline{D})^2}}{\overline{D}} \times 100$$

In which, $K_F$ is the freeze-thaw stability constant; n=m+1, the total times measuring the particle size during the experiment; $D_i$ means the particle size measured; $\overline{D}$ average particle size measured. The smaller $K_F$ indicates the oral nanocucurbitacin-contained emulsion is more stable; the larger, the more unstable.

## Experiments

### Experiment 1 Stability of the market-selling fat emulsions in freeze-thaw experiment and shaking experiment

### 1. Freeze-thaw stability

Market-selling fat emulsions products: 20% fat emulsion injection (20% LCT, 1.2%EPC, 2.2% Glycerol, (Sichuan Kelun Pharmaceutical Co., Ltd. Labeled as L-20-2.2), 30% fat emulsion injection (30% LCT, 1.2% EPC, 1.67% Glycerol, Xi'an Libang Pharmaceutical Co., Ltd. labeled as L-30-1.67), medium-chain and long-chain fat emulsion injection (10%LCT, 10% MCT, 1.2% EPC, 2.5% Glycerol, Germany/B.Braun Medical (Suzhou)) Company, labeled as LM-20-2.5), Structural fat emulsion injection (20% STG, 1.2% EPC, 2.2% Glycerol, Wuxi SSPC, labeled as STG-20-2.2) and fish oil fat emulsion injection (OMEGAVEN)containing 10% refine fish oil, 2.5% Glycerol, 1.2% EPC (labeled as F-10-2.5, SSPC); They are frozen in the temperature of -20 °C for 12-24h, followed by 3-6h in 20 °C as a cycle, then the particle sizes are measured.

In the freeze-thaw experiment, the inventors judge the stabilities by the freeze-thaw stability constant $K_F$, as shown in Table 1.

**Table 1 the appearance of the market-selling fat emulsions in the freeze-thaw experiments**

| L-20-2.2 | L-30-1.67 | LM-20-2.5 | STG-20-2.2 | F-10-2.5 |
|---|---|---|---|---|
| Few broken emulsions | Much broken emulsion with oil floating and separation | Oil floating | Oil floating and separation | Few broken emulsions |

From the Table, the market-selling fat emulsions cannot tolerate the freeze-thaw experiment.

**Experiment 2 the freeze-thaw stability of 20% fat emulsion injection improved by varied glycerol concentrations**

A bottle of market-selling 20% fat emulsion injection (L-20-2.2, comprising 20%LCT, 1.2% EPC, 2.2% Glycerol, Sichuan Kelun Pharmaceutical Co., Ltd.) is split into the 3 ml penicillin bottles with 2 ml each and 16 bottles in total; the glycerol is added into the bottles to render the glycerol (w/v%) in bottles as follows:2.2%, 5%, 7.5%, 10%, 20%, 30%, 40% and 50%, and two bottles for a concentration, which are further stirred for 20min in RT. Half of the bottles are for the freeze-thaw stability experiments; to ensure the glycerol uniformity in the fat emulsions, the inventors rotationally sterilize the other half of the emulsion in 100°C for 30 min, and carry out the stability experiments, as shown in Table 2-3.

**Table 2 results of the market-selling 20% fat emulsion injections (L-20) of different glycerol concentrations prior to sterilization**

| | 2.2% | | 5% | | 7.5% | | 10% | | 20% | | 30% | | 40% | | 50% | |
|---|---|---|---|---|---|---|---|---|---|---|---|---|---|---|---|---|
| | MD | CV | MD | CV | MD | CV | MD | CV | MD | CV | MD | CV | MD | CV | MD | CV |
| 0 | 296.6 | 0.560 | 299.5 | 0.549 | 293.6 | 0.602 | 299.9 | 0.515 | 313.1 | 0.519 | 293.9 | 0.481 | 302.0 | 0.523 | 323.5 | 0.656 |
| 1 | 458.6 | 0.535 | 253.9 | 0.593 | 298.0 | 0.632 | 292.7 | 0.542 | 293.4 | 0.558 | 292.6 | 0.583 | 297.9 | 0.584 | 303.2 | 0.614 |
| 2 | Oil floating | | 320.2 | 0.516 | 311.5 | 0.499 | 301.0 | 0.422 | 314.2 | 0.452 | 307.4 | 0.479 | 312.0 | 0.456 | 309.4 | 0.615 |
| 3 | Layer separation | | 310.9 | 0.515 | 311.8 | 0.565 | 320.9 | 0.519 | 315.3 | 0.474 | 318.4 | 0.546 | 317.3 | 0.540 | 313.8 | 0.611 |
| $K_f$ | $\infty$ | | 9.93 | | 3.07 | | 3.98 | | 3.38 | | 4.03 | | 2.90 | | 2.73 | |

**Table 3 results of the market-selling 20% fat emulsion injections (L-20) of different glycerol concentrations after sterilization**

|  | 2.2% | | 5% | | 7.5% | | 10% | | 20% | | 30% | | 40% | | 50% | |
|---|---|---|---|---|---|---|---|---|---|---|---|---|---|---|---|---|
|  | MD | CV | MD | CV | MD | CV | MD | CV | MD | CV | MD | CV | MD | CV | MD | CV |
| 0 | 305.9 | 0.586 | 304.2 | 0.542 | 272.4 | 0.590 | 295.4 | 0.548 | 305.4 | 0.542 | 286.6 | 0.612 | 318.6 | 0.517 | 308.0 | 0.644 |
| 1 | Layer separation | | 296.2 | 0.574 | 271.7 | 0.611 | 292.1 | 0.491 | 291.7 | 0.542 | 283.9 | 0.556 | 291.6 | 0.569 | 317.5 | 0.615 |
| 2 | Layer separation | | 360.2 | 0.408 | 291.3 | 0.374 | 300.2 | 0.322 | 295.4 | 0.311 | 299.5 | 0.363 | 304.4 | 0.392 | 313.3 | 0.628 |
| 3 | Layer separation | | 383.9 | 0.431 | 282.1 | 0.422 | 311.5 | 0.530 | 316.7 | 0.513 | 309.6 | 0.441 | 322.7 | 0.471 | 316.7 | 0.660 |
| $K_f$ | $\infty$ | | 12.71 | | 3.31 | | 2.83 | | 3.71 | | 4.05 | | 4.58 | | 1.38 | |

From the tables, the market-selling 20% fat emulsion injection (L-20) itself cannot tolerate the freeze-thaw experiment. When the emulsions increase their glycerol to 5%-50% (w/v), the emulsion injections are capable of tolerating at least three freeze-thaw cycles, however the 50% glycerol will cause the over-high CV, i.e. the uneven distribution of the particles, therefore the maximum concentration of the glycerol will be 40% (w/v %) for the coming experiments.

The freeze-thaw stability constant $K_F$s appear no notable change before and after the sterilization, which suggests the adding of the glycerol and the 20 min stirring in RT are sufficient for the uniform distribution of the glycerol in the fat emulsion injections. Additionally, the rotational sterilization of 30 min in 100 °C poses no effect on the stability of the fat emulsion injections.

**Experiment 3 the freeze-thaw stability of 30% fat emulsion injections improved by varied glycerol concentrations**

Similar to Experiment 2, adding glycerol to render the glycerol (w/v%) in market-selling 30% fat emulsion injection (L-30-1.67, 30% LCT, 1.2% EPC,1.67% glycerol,Xi'an Libang Pharmaceutical Co., Ltd) as follows: 2.2%, 5%, 7.5%, 10%, 15%, 20%, 30% and 40%, followed by further stirred for 20 min in RT.Half of the bottles are for the freeze-thaw stability experiments; the other half of the emulsion injections are rotationally sterilized in 100 °C for 30 min, and undergo the stability experiments. The results are shown in Table 4-5.

**Table 4 results of the market-selling 30% fat emulsion injections of different glycerol concentrations prior tosterilization**

| | 1.67% | | 2.5% | | 5% | | 7.5% | | 10% | | 15% | | 20% | | 30% | | 40% | |
|---|---|---|---|---|---|---|---|---|---|---|---|---|---|---|---|---|---|---|
| | MD | CV | MD | CV | MD | CV | MD | CV | MD | CV | MD | CV | MD | CV | MD | CV | MD | CV |
| 0 | 358.8 | 0.589 | 341.5 | 0.575 | 330.8 | 0.555 | 352.6 | 0.572 | 361.1 | 0.581 | 386.7 | 0.548 | 391.9 | 0.563 | 386.2 | 0.498 | 340.6 | 0.479 |
| 1 | Oil floating | | 441.8 | 0.569 | 418.0 | 0.571 | 324.9 | 0.607 | 357.0 | 0.443 | 376.0 | 0.587 | 392.1 | 0.617 | 394.3 | 0.553 | 368.6 | 0.539 |
| 2 | Layer separation | | 761.8 | 0.472 | 484.9 | 0.496 | 379.4 | 0.435 | 407.6 | 0.474 | 372.9 | 0.384 | 405.4 | 0.266 | 387.8 | 0.356 | 389.5 | 0.402 |
| 3 | Layer separation | | Oil floating | | 487.1 | 0.523 | 412.5 | 0.494 | 420.0 | 0.358 | 378.2 | 0.476 | 391.9 | 0.390 | 423.8 | 0.300 | 377.2 | 0.556 |
| $K_f$ | ∞ | | ∞ | | 17. 11 | | 10. 19 | | 8. 30 | | 1. 56 | | 1. 70 | | 4. 41 | | 5. 63 | |

**Table 5 results of the market-selling 30% fat emulsion injections of different glycerol concentrations after sterilization**

| | 1.67% | | 2.5% | | 5% | | 7.5% | | 10% | | 15% | | 20% | | 30% | | 40% | |
|---|---|---|---|---|---|---|---|---|---|---|---|---|---|---|---|---|---|---|
| | MD | CV | MD | CV | MD | CV | MD | CV | MD | CV | MD | CV | MD | CV | MD | CV | MD | CV |
| 0 | 395.8 | 0.567 | 386.0 | 0.582 | 443.1 | 0.504 | 306.0 | 0.598 | 361.1 | 0.581 | 380.5 | 0.581 | 399.5 | 0.554 | 332.8 | 0.549 | 330.0 | 0.612 |
| 1 | Layer separation | | Oil floating | | 437.0 | 0.656 | 392.2 | 0.475 | 444.1 | 0.433 | 415.6 | 0.525 | 427.9 | 0.506 | 399.1 | 0.332 | 331.2 | 0.456 |
| 2 | Layer separation | | Layer separation | | 537.3 | 0.369 | 416.1 | 0.517 | 409.9 | 0.501 | 416.1 | 0.526 | 402.2 | 0.507 | 394.7 | 0.317 | 408.5 | 0.397 |
| 3 | Layer separation | | Layer separation | | 657.7 | 0.373 | 324.5 | 0.595 | 416.7 | 0.439 | 411.8 | 0.463 | 395.1 | 0.435 | 405.5 | 0.480 | 408.5 | 0.571 |
| $K_f$ | $\infty$ | | $\infty$ | | 19.93 | | 14.64 | | 8.47 | | 4.21 | | 3.64 | | 8.82 | | 12.17 | |

From the tables, the market-selling 30% fat emulsion injection (L-30) itself cannot tolerate the freeze-thaw experiment. When the glycerol in the emulsions grows between 1.67% and 2.5% (w/v), the freeze-thaw issue is still not solved. When it reaches 7.5% (w/v), the emulsion injections are capable of tolerating at least three cycles of the freeze-thaw experiments; for the freeze-thaw stability constant $K_f$, it will go down at the early stage as the glycerol in the emulsions rises, and when it reaches 15% (w/v), the $K_f$ is the lowest, i.e. the fat emulsion injection is the most stable; and the $K_f$ will bounce as the glycerol increases, however the formulations can tolerate the experiments.

Compared the $K_f$s of the formulations before and after sterilization, the inventors find the $K_f$s after the sterilization are higher than that before the sterilization in different glycerol concentrations, which suggests the rotational sterilization of 30 min in 100°C influences the stability of the fat emulsion injections (L-30). It may be the unstable system of the over-high amount of the oil and the comparatively less phospholipids that makes it susceptible to the outer environment.

**Experiment 4 the freeze-thaw stability of market-selling medium-chain and long chain fat emulsion injections (ML-20) improved by varied glycerolconcentrations**

Similar to example 2, adding glycerol to render the glycerol (w/v%) in market-selling medium-chain and long chain fat emulsion injection (LM-20-2.5, 10% LCT, 10%MCT, 1.2% EPC,2.5% glycerol,Germany/B.Braun Medical (Suzhou)) Company) as follows: 2.5%, 5%, 7.5%, 10%, 20%, 30% and 40% , followed by further stirred for 20 min in RT. Half of the bottles are for the freeze-thaw stability experiments; the other half of the emulsion injections are rotationally sterilized in 100°C for 30 min, and undergo the stability experiments. The results are shown in Table 6-7.

Table 6 results of the market-selling medium-chain and long chain fat emulsion injections (ML-20) of different glycerol concentrations prior to sterilization

| | 2.5% | | 5% | | 7.5% | | 10% | | 15% | | 20% | | 30% | | 40% | |
|---|---|---|---|---|---|---|---|---|---|---|---|---|---|---|---|---|
| | MD | CV | MD | CV | MD | CV | MD | CV | MD | CV | MD | CV | MD | CV | MD | CV |
| 0 | 284.4 | 0.550 | 286.7 | 0.574 | 284.5 | 0.519 | 294.4 | 0.542 | 293.6 | 0.549 | 294.0 | 0.463 | 296.8 | 0.510 | 300.7 | 0.506 |
| 1 | Oil floating | | 375.4 | 0.519 | 273.2 | 0.560 | 295.9 | 0.453 | 281.5 | 0.517 | 290.7 | 0.432 | 282.9 | 0.509 | 268.7 | 0.544 |
| 2 | Layer separation | | 396.9 | 0.545 | 299.6 | 0.432 | 303.8 | 0.439 | 297.6 | 0.391 | 282.6 | 0.300 | 291.2 | 0.300 | 307.3 | 0.351 |
| 3 | Layer separation | | 417.0 | 0.544 | 310.0 | 0.526 | 317.4 | 0.386 | 298.0 | 0.392 | 302.1 | 0.502 | 296.5 | 0.414 | 297.4 | 0.429 |
| $K_f$ | $\infty$ | | 15.57 | | 5.57 | | 3.47 | | 2.63 | | 2.76 | | 2.23 | | 5.81 | |

**Table 7 results of the market-selling medium-chain and long chain fat emulsion injections (ML-20) of different glycerol concentrations after sterilization**

| | 2.5% | | 5% | | 7.5% | | 10% | | 15% | | 20% | | 30% | | 40% | |
|---|---|---|---|---|---|---|---|---|---|---|---|---|---|---|---|---|
| | MD | CV | MD | CV | MD | CV | MD | CV | MD | CV | MD | CV | MD | CV | MD | CV |
| 0 | 272.7 | 0.511 | 292.0 | 0.542 | 294.5 | 0.516 | 312.4 | 0.498 | 282.4 | 0.511 | 296.8 | 0.542 | 297.0 | 0.504 | 321.1 | 0.429 |
| 1 | Layer separation | | Oil floating | | 272.7 | 0.554 | 274.4 | 0.493 | 275.5 | 0.384 | 269.2 | 0.514 | 231.0 | 0.584 | 272.6 | 0.581 |
| 2 | Layer separation | | Layer separation | | 300.9 | 0.418 | 306.2 | 0.477 | 284.7 | 0.400 | 299.1 | 0.362 | 287.2 | 0.377 | 309.4 | 0.374 |
| 3 | Layer separation | | Layer separation | | 312.4 | 0.397 | 288.6 | 0.554 | 307.1 | 0.469 | 313.4 | 0.447 | 314.9 | 0.417 | 314.1 | 0.417 |
| $K_f$ | $\infty$ | | $\infty$ | | 5.65 | | 5.84 | | 4.76 | | 6.27 | | 12.82 | | 7.12 | |

From the tables, the market-selling medium-chain and long chain fat emulsion injections (ML-20) itself before and after the sterilization cannot tolerate the freeze-thaw experiments.

For the ML-20 before the sterilization, when the glycerol in the emulsions grows between 2.5% and 5% (w/v), the $K_f$ will also fall down at first and bounce back, similar to L-20. When the glycerol is 15% (w/v), $K_f$ is the lowest, and the emulsion injection is the most stable.

For the ML-20 after the sterilization, 5% (w/v) glycerol cannot solve the stability issue. The formulations will have layer separation after three freeze-thaw cycles, suggesting not only the glycerol, but the firmness of the emulsion membrane influences its stability. The result is possibly caused by the weak and loose ML-20 membrane formed in the rotating sterilization of 100°C for 30 min. as the glycerol increases, the formulation will tolerate the experiment again, however the $K_f$ will not witness the trend of falling at first and bouncing back finally, even for 30% glycerol, $K_f$ has a large increase, duce to the alternation of the emulsion membrane.

Similar results are found in the fish oil fat emulsion injection (OMEGAVEN) containing 10% refine fish oil, 2.5% Glycerol and 1.2% EPC (labeled as F-10-2.5, SSPC), which proves the glycerol concentration shall be greater than 5%.

**Experiment 5 the freeze-thaw stability of the market-selling structural fat emulsion injections improved by varied glycerol concentrations**

Similar to example 2, adding glycerol to render the glycerol (w/v%) in market-selling structural fat emulsion injections (STG-20-2.2, 20%STG, 1.2%EPC, 2.2% glycerol, Wuxi SSPC) as follows: 2.2%, 5%, 7.5%, 10%, 15%, 20%, 30% and 40%, followed by further stirred for 20 min in RT. Half of the bottles are for the freeze-thaw stability experiments; the other half of the emulsion injections are rotationally sterilized in 100 °C for 30 min, and undergo the stability experiments. The results are shown in Table 8-9.

**Table 8 results of the market-selling structural fat emulsion injections (STG-20-2.2) of different glycerol concentrations prior to sterilization**

| | 2.2% | | 5% | | 7.5% | | 10% | | 15% | | 20% | | 30% | | 40% | |
|---|---|---|---|---|---|---|---|---|---|---|---|---|---|---|---|---|
| | MD | CV | MD | CV | MD | CV | MD | CV | MD | CV | MD | CV | MD | CV | MD | CV |
| 0 | 267.0 | 0.521 | 263.0 | 0.418 | 245.6 | 0.600 | 231.8 | 0.565 | 278.4 | 0.464 | 264.1 | 0.570 | 272.8 | 0.554 | 275.6 | 0.570 |
| 1 | Layer separation | | Oil floating | | 227.7 | 0.529 | 246.5 | 0.527 | 255.7 | 0.393 | 256.3 | 0.479 | 262.3 | 0.447 | 274.0 | 0.507 |
| 2 | Layer separation | | Layer separation | | 303.6 | 0.472 | 273.1 | 0.351 | 284.3 | 0.421 | 283.3 | 0.530 | 289.3 | 0.402 | 273.5 | 0.353 |
| 3 | Layer separation | | Layer separation | | 308.9 | 0.417 | 289.6 | 0.530 | 283.0 | 0.502 | 286.9 | 0.435 | 296.0 | 0.453 | 290.2 | 0.429 |
| $K_f$ | $\infty$ | | $\infty$ | | 15.07 | | 9.98 | | 4.85 | | 5.43 | | 5.48 | | 2.86 | |

**Table 9 results of the market-selling structural fat emulsion injections (STG-20-2.2) of different glycerol concentrations after sterilization**

|  | 2.2% | | 5% | | 7.5% | | 10% | | 15% | | 20% | | 30% | | 40% | |
|---|---|---|---|---|---|---|---|---|---|---|---|---|---|---|---|---|
|  | MD | CV | MD | CV | MD | CV | MD | CV | MD | CV | MD | CV | MD | CV | MD | CV |
| 0 | 279.5 | 0.519 | 276.2 | 0.528 | 247.8 | 0.552 | 251.2 | 0.581 | 275.0 | 0.509 | 276.4 | 0.531 | 276.3 | 0.507 | 282.5 | 0.520 |
| 1 | Layer separation | | Layer separation | | 238.1 | 0.545 | 253.4 | 0.520 | 295.9 | 0.387 | 290.7 | 0.494 | 280.0 | 0.514 | 293.2 | 0.450 |
| 2 | Layer separation | | Layer separation | | 292.5 | 0.398 | 279.1 | 0.350 | 280.0 | 0.267 | 284.7 | 0.416 | 282.1 | 0.349 | 275.5 | 0.534 |
| 3 | Layer separation | | Layer separation | | 290.4 | 0.446 | 298.0 | 0.230 | 291.2 | 0.426 | 291.3 | 0.442 | 289.5 | 0.426 | 283.1 | 0.403 |
| $K_f$ | $\infty$ | | $\infty$ | | 10.59 | | 8.26 | | 3.39 | | 2.42 | | 1.97 | | 2.57 | |

From the tables, the market-selling structural fat emulsion injections (STG-20) before and after sterilization cannot tolerate the experiments, and when the glycerol is 5% (w/v), the formulations before and after the sterilization are still not capable of tolerating the experiments, suggesting the essential role of the composition in the oil phase of the emulsions.

Contrary to the L-10, L-20, L-30 and ML-20, the $K_f$s of the STG-20 in different glycerol concentration ranging 2.2%-40% (w/v) before and after sterilization do not experience the falling and rising trend, and are constantly decreasing as the glycerol grows.

Overall, when the glycerol is greater than 7.5% (w/v), the formulations tolerate at least three cycles of the freeze-thaw experiments.

**Experiment 6 shaking stability**

The market-selling fat emulsions of different glycerol concentrations are destroyed by the shaking intensity of 100/min, and the results are shown in table 10-14, in which:"-" no aggregate, uniform; "+"aggregate /or small oil drop; "++" big oil drop/or many small oil drops;"+++" layers separation.

**Table 10 Shaking stability of the market-selling fat emulsions (L-20-2.2) of different glycerol concentrations**

| Shaking Period (h) | L-20-2.2 | L-20-3.0 | L-20-5.0 | L-20-10 | L-20-20 |
|---|---|---|---|---|---|
| 0 | - | - | - | - | - |
| 5 | + | - | - | - | - |
| 12 | ++ | + | - | - | - |
| 24 | +++ | + | - | - | - |
| Note: L-20-2.2 emulsions of 3%, 5%,10 % and 20% glycerol are labeled as L-20-3.0, L-20-5.0, L-20-10 and L-20-20. | | | | | |

**Table 11 shaking stability of the L-30-1.67 of different glycerol concentrations**

| Shaking Period (h) | L-30-1.67 | L-30-3.0 | L-30-5.0 | L-30-10 | L-30-20 |
|---|---|---|---|---|---|
| 0 | - | - | - | - | - |
| 5 | + | - | - | - | - |
| 12 | +++ | + | - | - | - |
| 24 | +++ | + | + | - | - |
| Note: L-30-1.67 emulsions of 3%, 5%,10 % and 20% glycerol are labeled as L-30-3.0, L-30-5.0, L-30-10 and L-30-20. | | | | | |

**Table 12 shaking stability of the LM-20-2.5 with different glycerolconcentrations**

| Shaking Period (h) | LM-20-2.5 | LM-20-3.0 | LM-30-5.0 | LM-20-10 | LM-20-20 |
|---|---|---|---|---|---|
| 0 | - | - | - | - | - |
| 5 | + | - | - | - | - |
| 12 | ++ | - | - | - | - |
| 24 | +++ | + | - | - | - |
| Note: LM-20-2.5 emulsions of 3%, 5%, 10 % and 20% glycerol are labeled as LM-20-3.0, LM-20-5.0, LM-20-10 and LM-20-20. | | | | | |

**Table 13 shaking stability of the STG-20-2.2of different glycerol concentrations**

| Shaking Period (h) | STG-20-2.2 | STG-20-3.0 | STG-20-5.0 | STG-20-10 | STG-20-20 |
|---|---|---|---|---|---|
| 0 | - | - | - | - | - |
| 5 | + | - | - | - | - |
| 12 | +++ | + | - | - | - |
| 24 | +++ | + | + | - | - |
| Note:STG-20-2.2 emulsions of 3%, 5%,10 % and 20% glycerol are labeled as STG -20-3.0, STG -20-5.0, STG -20-10 and STG -20-20. | | | | | |

**Table 14 shaking stability of the F-10-2.5of different glycerolconcentrations**

| Shaking Period (h) | F-10-2.5 | F-10-3.0 | F-10-5.0 | F-10-10 | F-10-20 |
|---|---|---|---|---|---|
| 0 | - | - | - | - | - |
| 5 | + | - | - | - | - |
| 12 | + | - | - | - | - |
| 24 | ++ | - | - | - | - |
| Note:F-20-2.2 emulsions of 3%, 5%,10 % and 20% glycerol are labeled as F -20-3.0, F - 20-5.0, F -20-10 and F -20-20. | | | | | |

**Experiment 7 the making of the 10% LCT fat emulsion (L-10-2.5) and the freeze-thaw stability investigation**

1. The making of 10% LCT fat emulsion injection (L-10)

The self-made fat emulsion injection formula is shown in table 15.

**Table 15 self-made fat emulsion injection formula**

| ingredients | content (w/v) |
|---|---|
| Soy bean oil LCT | 10% |
| EPC | 1.2% |
| Sodium oleate | 0.02% |
| Glycerol | 2.5% |
| Sterile injection water | Up to 100% |

Process:

1) Mix and heat the LCT and EPC of the formula content to obtain the oil phase for use;
2) The water phase is made by mixing the sodium oleate, glycerol and sterile injection water of the formula content for use;
3) Heat the two phases to 65 °C, and slowly add the water phase into the oil phase under the magnetic stirring, followed by further 20 min stirring , then obtain the pre-emlusion ;
4) adjust the pre-emulsion pH to 8.5 by 10 mM NaOH, followed by 5 times homogenization by a Microfluidizer under 14000 psi, and then dilute the solution to 100ml by sterile injection water. Filter the solution by 0.45um micro filter to obtain the emulsion. The emulsion is put into 3 ml penicillin bottles with 2ml in each and the nitrogen is filled into the bottles plugged and sealed by aluminum caps; The bottles are sterilized in 121°C for 10 min, followed by cold water sprinkling to the RT, the emulsion product is obtained.

## 2. freeze-thaw stability investigation

Adding glycerol into 6 bottles of the said renders the glycerol (w/v) as follows: 3%, 5%, 10%, 20%, 30% and 40%, followed by 20min magnetic stirring, and the injections are obtained.

The self-made fat emulsion injections of different glycerol concentrations are investigated by freeze-thaw stability experiment, and the results are shown in table 16.

**Table 16 results of the freeze-thaw stability of the self-made fat emulsion injections of different glycerol concentrations**

| | 2.5% | | 3% | | 5% | | 10% | | 20% | | 30% | | 40% | |
|---|---|---|---|---|---|---|---|---|---|---|---|---|---|---|
| | MD | CV | MD | CV | MD | CV | MD | CV | MD | CV | MD | CV | MD | CV |
| 0 | 243.60 | .351 | 245.1 | 0.332 | 231.4 | 0.460 | 241.6 | 0.381 | 246.1 | 0.427 | 231.7 | 0.449 | 249.1 | 0.379 |
| 1 | Small concentration of floating oil | | 237.0 | 0.414 | 239.2 | 0.413 | 236.7 | 0.449 | 238.8 | 0.426 | 243.8 | 0.412 | 248.6 | 0.449 |
| 2 | Large concentration of floating oil | | 214.5 | 0.532 | 240.2 | 0.258 | 225.3 | 0.469 | 232.2 | 0.530 | 232.3 | 0.458 | 235.8 | 0.518 |
| 3 | Layer separation | | 222.7 | 0.486 | 225.8 | 0.565 | 233.0 | 0.477 | 239.3 | 0.165 | 243.2 | 0.420 | 248.1 | 0.411 |
| $K_f$ | | | 6.00 | | 2.91 | | 2.93 | | 2.37 | | 2.80 | | 2.61 | |

From the results, the fat emulsion of 2.5% glycerol does not tolerate the freeze-thaw experiments, however, when the glycerol is 3%, or 5%, or 10%, or 20%, or 30% or 40%, the emulsions tolerate the experiments. (Note: the emulsions of 30% and 40% glycerol after 2 years storage exhibit small concentration of layers separation, and more severe in 40%, but no significant increase is found in particle size.)

Base on that, the inventors continue the survey on the fat emulsions stability of different oil concentrations (10%, 7.5%, 5% and 2.5%) (Fixed oil/phospholipids ratios), the formula is shown in table 17.

**Table 17 Emulsion formula of different oil concentrations**

| Ingredient | amount | amount | amount | amount |
|---|---|---|---|---|
| Soybean Oil | 10g | 7.5 g | 5.0 g | 2.5g |
| EPC | 1.2 g | 0.9g | 0.6 g | 0.3g |
| Glycerol | 3.0g | 3.0g | 3.0g | 3.0g |
| Injection Water | Up to 100 mL | Up to 100 mL | Up to 100 mL | Up to 100 mL |

The making process is similar to the previous experiments, the results show the emulsions can tolerate many cycles of freeze-thaw destruction.

**Experiment 8 the effect of different glycerol concentrations on the freeze-thaw stability of the fat emulsion of different pHs before and after sterilization**

The 10% LCT fat emulsion injections of different pHs are made by the process similar to the Experiment 7 with different glycerol concentrations (Because the results of Experiment 7 show that 2.5% glycerol contained fat emulsion cannot tolerate freeze-thaw experiment and is discarded.). The formulations will be investigated about its freeze-thaw stability, and the results are shown in table 18.

Table 18 effect of different glycerol concentrations on the freeze-thaw stability of the fat emulsion of different pH before and after sterilization

| | | 3% | | 10% | | 15% | | 20% | | 30% | | 40% | |
|---|---|---|---|---|---|---|---|---|---|---|---|---|---|
| | | MD | CV | MD | CV | MD | CV | MD | CV | MD | CV | MD | CV |
| Em6.5 | 0 | 277.5 | 0.503 | 271.3 | 0.380 | 266.9 | 0.451 | 269.5 | 0.411 | 285.2 | 0.437 | 289.4 | 0.418 |
| | 1 | 240.3 | 0.440 | 266.8 | 0.335 | 257.2 | 0.426 | 255.8 | 0.336 | 289.1 | 0.408 | 281.2 | 0.453 |
| | 2 | 236.8 | 0.535 | 263.5 | 0.401 | 245.3 | 0.430 | 256.8 | 0.429 | 271.6 | 0.413 | 279.6 | 0.416 |
| | 3 | 237.7 | 0.495 | 241.1 | 0.426 | 251.8 | 0.461 | 253.6 | 0.462 | 268.7 | 0.450 | 260.2 | 0.493 |
| | | | | | | | | | | | | | |
| Em7.5 | 0 | 255.4 | 0.491 | 242.0 | 0.440 | 244.6 | 0.423 | 249.1 | 0.439 | 268.5 | 0.444 | 265.5 | 0.346 |
| | 1 | 239.3 | 0.499 | 239.9 | 0.397 | 237.3 | 0.391 | 240.8 | 0.431 | 228.9 | 0.423 | 234.9 | 0.467 |
| | 2 | 259.9 | 0.494 | 233.8 | 0.442 | 247.4 | 0.530 | 226.8 | 0.499 | 227.9 | 0.470 | 241.2 | 0.482 |
| | 3 | 246.5 | 0.489 | 239.2 | 0.482 | 230.9 | 0.554 | 241.9 | 0.502 | 241.1 | 0.493 | 254.9 | 0.477 |
| | | | | | | | | | | | | | |
| Em8.0 | 0 | 240.7 | 0.376 | 254.6 | 0.311 | 267.2 | 0.400 | 244.5 | 0.405 | 235.3 | 0.374 | 264.1 | 0.499 |
| | 1 | 231.8 | 0.349 | 236.0 | 0.500 | 225.9 | 0.516 | 244.6 | 0.439 | 235.8 | 0.383 | 235.9 | 0.407 |
| | 2 | 215.5 | 0.526 | 228.4 | 0.517 | 237.9 | 0.417 | 234.5 | 0.569 | 233.6 | 0.443 | 233.9 | 0.475 |
| | 3 | 222.8 | 0.518 | 237.4 | 0.484 | 238.0 | 0.384 | 250.8 | 0.501 | 241.4 | 0.458 | 241.0 | 0.490 |
| | | | | | | | | | | | | | |
| Em8.5 | 0 | 245.1 | 0.332 | 231.4 | 0.460 | 241.6 | 0.381 | 246.1 | 0.427 | 231.7 | 0.449 | 249.1 | 0.379 |
| | 1 | 237.0 | 0.414 | 239.2 | 0.413 | 236.7 | 0.449 | 238.8 | 0.426 | 243.8 | 0.412 | 248.6 | 0.449 |
| | 2 | 214.5 | 0.532 | 240.2 | 0.258 | 225.3 | 0.469 | 232.2 | 0.530 | 232.3 | 0.458 | 235.8 | 0.518 |
| | 3 | 222.7 | 0.486 | 225.8 | 0.565 | 233.0 | 0.477 | 239.3 | 0.165 | 243.2 | 0.420 | 248.1 | 0.411 |
| | | | | | | | | | | | | | |
| Note: Em6.5, Em7.5, Em8.0 and Em8.5 represent the emulsions of pH 6.5, 7.5, 8.0 and 8.5 respectively. | | | | | | | | | | | | | |

From the table, when glycerol is greater than 3%, the fat emulsions of pH6.5-8.5 tolerate the freeze-thaw destruction. (Note: the fat emulsions in pH 6 are unstable.)

**Experiment 9 glycerol reduces the pH change of the fat emulsion before and after sterilization**

The experiment is to make a freeze-thaw resistant fat emulsion. The basic formula is made by changing the glycerol to 5% (w/v) based on the market-selling fat emulsion formula, as shown in table 19.

**Table 19 basic formula of the fat emulsions**

| Ingredients | contents (w/v) |
|---|---|
| E80 | 1.2% |
| LCT | 20% |
| Oleic acid | 0.02% |
| Glycerol | 2.5% |
| Sterile injection water | Up to 100% |

Process: the oil phase is made up of E80, LCT and oleic acid in their contents; and the water phase is made up of glycerol and 85% (v/v) sterile injection water, and the two phases are heated to 65 °C. When the materials in the oil phase fully dissolve, then the water phase is slowly added into the oil phase stirred by magnetic stirrer. Continue the stirring for 20min to obtain the pre-emulsion. The pre-emulsion is homogenized five times by a microfluider, diluted by the sterile injection water to 1000 ml, filtered by 0.22um microfilters and pHs are measured; the pre-emulsion is divided into seven parts (glycerol adjusted to 2.5%, 5.0%, 10%, 15%, 20%, 30% and 40%), and pHs are adjusted by 10 mM NaOH to pH 7.5, 8.0 and 8.5, as Em 7.5, Em8.0, Em8.5, which are bottled into 7ml penicillin bottles with 4ml in each. The bottles of the pre-emulsion is blended and filled with nitrogen and capped and sealed by aluminum caps; half of the formulations is labeled as pre-formulations without sterilization; the other half are for 10min in 121 °C, labeled as post-formulations, cooled by cold water sprinkling to RT. Following these steps, the emulsions are obtained.

**Table 20 effect of glycerol on the pHs of the fat emulsions of different pHs before and after sterilization**

| | | 2.5% | 5% | 10% | 15% | 20% | 30% | 40% |
|---|---|---|---|---|---|---|---|---|
| Em7.5 | Before | 7.49 | 7.43 | 7.32 | 7.28 | 7.27 | 7.23 | 7.20 |
| | After | 6.34 | 6.43 | 6.48 | 6.50 | 6.38 | 6.41 | 6.31 |
| | $\Delta$pH | 1.35 | 1.00 | 0.84 | 0.78 | 0.89 | 0.82 | 0.89 |
| Em8.0 | Before | 8.00 | 8.03 | 7.87 | 7.72 | 7.93 | 7.87 | 7.79 |
| | After | 6.67 | 6.75 | 7.19 | 6.91 | 7.08 | 6.91 | 6.74 |
| | $\Delta$pH | 1.33 | 0.98 | 0.68 | 0.81 | 0.85 | 0.96 | 1.05 |
| Em8.5 | Before | 8.51 | 8.58 | 8.31 | 8.38 | 8.46 | 8.57 | 8.41 |
| | After | 6.80 | 7.05 | 7.17 | 7.35 | 7.35 | 7.38 | 7.10 |
| | $\Delta$pH | 1.71 | 1.33 | 1.14 | 1.03 | 1.11 | 1.19 | 1.31 |

Evidently, the glycerol increase will reduce the pH changes before and after sterilization, and the pH change is the smallest for the 10-20% glycerol emulsions.

Unexpectedly inventors find that the pH changes reduce as the glycerol increases , which is beneficial for the industrialized control and batch control; when the glycerol is too high (more than 20%), pH changes will increase, the optimal concentrations of glycerol is 5%-15%.

**Experiment 10 compatibility of the metal ions with fat emulsions**

Similar to Experiment 2, the adding of the glycerol renders the structural fat emulsion injections (STG-20-2.2, 20%STG, 1.2%EPC , 2.2% glycerol, Wuxi SSPC) containing glycerol as follows 2.2%, 5%, 7.5%, 10%, 15%, 20%, 30% and 40%, followed by magnetic stirring for 20min. half of the formulations are for the freeze-thaw stability experiments, and the other half are for testing the effect of $CaCl_2$ on the freeze-thaw stability of the medium-chain and long chain fat emulsions of different glycerol concentrations.

Based on the formula of the market-selling medium-chain and long-chain fat emulsion injections (ML-20), the formula are added with glycerol to make the glycerol fat emulsions as follows: 2.5%, 5.0% and 10.0% (w/v), and the fat emulsions are added with 200ul $CaCl_2$ to get the concentrations as follows: 0.1 g/L, 0.3 g/L, 0.5g/L, 1.0 g/L and 1.5 g/L, followed by magnetic stirring for 20 min in RT. Then the freeze-thaw stability experiment is carried out and the results are shown in table 21-25.

**Table 21 effect of 0.1g/L $CaCl_2$ on the freeze-thaw stability of the market-selling 20% medium-chain and long-chain fat emulsion injections of different glycerol concentrations**

| Freeze-thaw cycle(s) | 2.5% | | 5% | | 10% | |
|---|---|---|---|---|---|---|
| | MD | CV | MD | CV | MD | CV |
| 0 | 321.2 | 0.481 | 326.3 | 0.483 | 323.8 | 0.540 |
| 1 | Oil floating | - | 362.3 | 0.510 | 328.3 | 0.468 |
| 2 | Oil and water separation | | 397.2 | 0.493 | 338.6 | 0.503 |

**Table 22 effect of 0.3g/L $CaCl_2$ on the freeze-thaw stability of the market-selling 20% medium-chain and long-chain fat emulsion injections of different glycerol concentrations**

| Freeze-thaw cycle(s) | 2.5% | | 5% | | 10% | |
|---|---|---|---|---|---|---|
| | MD | CV | MD | CV | MD | CV |
| 0 | 321.2 | 0.481 | 326.3 | 0.483 | 323.8 | 0.540 |
| 1 | Oil floating | - | 358.1 | 0.506 | 331.1 | 0.477 |
| 2 | Oil and water separation | | 388.6 | 0.485 | 340.3 | 0.511 |

**Table 23 effect of 0.5g/L $CaCl_2$ on the freeze-thaw stability of the market-selling 20% medium-chain and long-chain fat emulsion injections of different glycerol concentrations**

| | 2.5% | | 5% | | 10% | |
|---|---|---|---|---|---|---|
| | MD | CV | MD | CV | MD | CV |
| 0 | 321.2 | 0.481 | 326.3 | 0.483 | 323.8 | 0.540 |
| 1 | 400.6 | 0.282 | 359.7 | 0.500 | 324.5 | 0.507 |
| 2 | Oil and water separation | | 373.9 | 0.492 | 336.3 | 0.483 |
| 3 | Oil and water separation | | 437.2 | 0.465 | 331.7 | 0.557 |

**Table 24 effect of 1.0 g/L $CaCl_2$ on the freeze-thaw stability of the market-selling 20% medium-chain and long-chain fat emulsion injections of different glycerol concentrations**

| | 2.5 % | | 5% | | 10% | |
|---|---|---|---|---|---|---|
| | MD | CV | MD | CV | MD | CV |
| 0 | 318.2 | 0.458 | 324.8 | 0.532 | 328.4 | 0.501 |
| 1 | 335.9 | 0.146 | 339.8 | 0.484 | 384.5 | 0.522 |
| 2 | 447.6 | 0.569 | 385.5 | 0.442 | 329.2 | 0.492 |
| 3 | Oil and water separation | | 415.1 | 0.473 | 338.4 | 0.435 |

**Table 25 effect of 1.5g/L CaCl$_2$ on the freeze-thaw stability of the market-selling 20% medium-chain and long-chain fat emulsion injections of different glycerol concentrations**

| | 2.5% | | 5% | | 10% | |
|---|---|---|---|---|---|---|
| | MD | CV | MD | CV | MD | CV |
| 0 | 326.2 | 0.492 | 321.1 | 0.471 | 327.8 | 0.496 |
| 1 | 329.2 | 0.465 | 345.7 | 0.414 | 343.2 | 0.463 |
| 2 | 433.9 (PFAT-5unqualified) | 0.548 | 383.4 | 0.510 | 333.3 | 0.548 |
| 3 | 522.6 (PFAT-5unqualified) | 0.532 | 431.6 (PFAT-5 unqualified) | 0.537 | 376.4 | 0.490 |

Evidently there are some problems with the mixing of the market-selling emulsions and the Ca$^{2+}$. The low-concentration Ca salt is more likely to induce the instability, and the stability of the high-concentration Ca$^{2+}$ and the fat emulsion are better. The 2.5% glycerol emulsion can only tolerate one cycle, but the 5% glycerol emulsion can tolerate two cycles, macroparticle will appear in the third cycle; 10% glycerol will completely eliminate the effect of the Ca$^{2+}$.

Inventors mix the fat emulsions with MgCl$_2$ (0.05%, 0.1% and 0.2%, g/ml) and find the similar problems, i.e. the low-concentration MgCl$_2$ enlarges the particles, while the particles in the high-concentration of MgCl$_2$ are small. 10% glycerol will completely eliminate the effect of the MgCl$_2$.

**Experiment 11 the freeze-thaw stability of market-selling dexamethasone palmitateinj ection**

Four market-selling dexamethasone palmitate injections (LIMETHASON, Mitsubishi Pharma(Guangzhou)Co., Ltd. Product Batch No.V095;Import Drug License No:H20120480; Sub packaging Batch No. LI131107) are added glycerol to make its concentrations in the injections as follows: 2.2% (w/v, glycerol-added 0, market-selling formulation), 3.0% (w/v, glycerol-added 0.8%), 4.5% (w/v, glycerol-added 2.3%) and 6.0% (w/v, glycerol-added 3.8%), followed by the freeze-thaw stability experiment, and the results are shown in Table 26.

**Table 26 freeze-thaw stability results**

| Freeze-thaw cycle(s) | 2.2%glycerol | 3.0%glycerol | 4.5% | 6.0% |
|---|---|---|---|---|
| 0 | 196.0 | 193.6 | 198.6 | 191.3 |
| 1 | 1071.1/oil floating | 1357.9/oil floating | 201.3 | 198.0 |
| 2 | - | - | 225.2 | 200.1 |
| 3 | - | - | 222.0 | 196.5 |

From the table, the dexamethasone palmitate emulsions of glycerol more than 4.5% tolerate the freeze-thaw experiments, while the 3% glycerol emulsion fails. However, the 10% LCT emulsion of glycerol 3% in the Experiment 7 tolerates the experiment, i.e. the drug alters the oil phase and makes it sensitive to the freeze-thaw destruction, which can be solved by further increasing the glycerol.

The dexamethasone palmitate injection is to treat the newborn *West* syndrome (J Child Neurol 2000; 15:702-704 and Brain and Development 2007, 29 (7) : 421-424). Because of the child' s sensitivity to the environment and the thin capillary vessels of the newborns, the quality of the fat emulsion is extremely important, any macroparticles /or small changes in the formulation quality will cause serious issues.

**Experiment 12 oleic acid increases the capability of dexamethasone palmitate injections to resist the freeze-thaw experiment**

Based on the market-selling dexamethasone palmitate injection formula (4.0 mg dexamethasone palmitate, 100mg soybean oil, 12mg EPC and 22.1mg thick glycerol in 1ml) and Cleviprex formula (containing 0.03g oleic acid), the formula of dexamethasone palmitate injections containing oleic acid of different glycerol concentrations is shown in table 27.

**Table 27 formula of dexamethasone palmitate injections of different glycerolconcentrations**

| Ingredients | glycerol (w/v) | | | | |
|---|---|---|---|---|---|
| | 2.2% | 3.0% | 5.0% | 8.0% | 10.0% |
| dexamethasone palmitate | 0.4g | 0.4g | 0.4g | 0.4g | 0.4g |
| EPC | 1.2 g | 1.2 g | 1.2 g | 1.2 g | 1.2 g |
| LCT | 10 g | 10 g | 10 g | 10g | 10g |
| Oleic acid | 0.03g | 0.03g | 0.03g | 0.03g | 0.03g |
| Glycerol | 2.21g | 3.0g | 5.0 g | 8.0 g | 10.0 g |
| Sodium Hydroxide | proper | proper | proper | proper | proper |
| Inj ection Water | Up to 100 mL | Up to 100 mL | Up to 100 mL | Up to 100 mL | Up to100mL |

The process is similar to that of Experiment 9, and pH is 8.5.

The results of the freeze-thaw stability of the dexamethasone palmitate injections of different glycerol concentrations are shown in Table 28.

**Table 28 freeze-thaw stability results**

| Cycle(s) | Glycerol (w/v) | | | | | | | | | |
|---|---|---|---|---|---|---|---|---|---|---|
| | 2.21% | | 3.0% | | 5.0% | | 8.0% | | 10.0% | |
| | MD | CV | MD | CV | MD | CV | MD | CV | MD | CV |
| 0 | 271.0 | 0.422 | 258.6 | 0.376 | 255.0 | 0.320 | 232.8 | 0.309 | 240.9 | 0.343 |
| 1 | 1042.8 | 0.813 | 262.8 | 0.413 | 256.7 | 0.349 | 234.7 | 0.227 | 236.7 | 0.232 |
| 2 | Oil floating | -- | 261.4 | 0.378 | 252.4 | 0.358 | 232.8 | 0.451 | 237.5 | 0.433 |
| 3 | separation | -- | 289.4 | 0.481 | 262.4 | 0.458 | 235.8 | 0.552 | 237.6 | 0.500 |

Compared with the result of Experiment 11, i.e. the dexamethasone palmitate injections without oleic acid, the oleic acid can enhance the freeze-thaw resistance to enable the 3.0% glycerol to resist the freeze-thaw experiments.

**Experiment 13 effect of glycerol on the stability of the dexamethasone palmitate injections**

1. The making of the dexamethasone palmitate injections of different glycerol concentrations

Based on the market-selling dexamethasone palmitate injection formula (4.0 mg dexamethasone palmitate , 100mg soybean oil, 12mg EPC and 22.1mg thick glycerol in 1ml), the formula of dexamethasone palmitate injections containing oleic acid of different glycerolconcentrations is shown in table 29.

**Table 29 formula of dexamethasone palmitate injections of different glycerol concentrations**

| Ingredients | Glycerol (w/v) | | | | |
|---|---|---|---|---|---|
| | 2.2% | 3.0% | 5.0% | 8.0% | 10.0% |
| dexamethasone palmitate | 0.4g | 0.4g | 0.4g | 0.4g | 0.4g |
| EPC | 1.2 g | 1.2 g | 1.2 g | 1.2 g | 1.2 g |
| LCT | 10 g | 10 g | 10 g | 10g | 10g |
| Oleic acid | 0.03g | 0.03g | 0.03g | 0.03g | 0.03g |
| Glycerol | 2.21g | 3.0g | 5.0 g | 8.0 g | 10.0 g |
| Sodium Hydroxide | proper | proper | proper | proper | proper |
| Injection Water | Up to 100 mL | Up to 100 mL | Up to 100 mL | Up to 100 mL | Up to 100mL |

The process is similar to the Experiment 7.

Based on the formula of the Experiment 11, the dexamethasone palmitate has ester bond, which is easily degraded in the basic condition, reduces the pH by some 1 after sterilization. Therefore the pH prior to the sterilization is regulated to 8.06, 8.51, 9.03, 9.50 and 10.10 for 10 days in 60 °C, the related substances/ degradation products are decreasing under the glycerol less than 10%, the result is shown in Table 30.

**Table 30 related substances/degradation products of dexamethasone palmitate injections of different glycerol concentrations**

| pH | Related Substances/Degradation Products (%) | | | | |
|---|---|---|---|---|---|
| | 2.2%glycerol | 3.0%glycerol | 5.0%glycerol | 8.0%glycerol | 10.0%glycerol |
| 8.06 | 4.5 | 4.7 | 4.2 | 3.8 | 3.3 |
| 8.51 | 5.3 | 5.1 | 4.9 | 4.2 | 3.5 |
| 9.03 | 7.1 | 6.3 | 5.1 | 4.3 | 4.2 |
| 9.50 | 11.5 | 8.2 | 7.3 | 5.5 | 5.6 |
| 10.10 | 18.6 | 13.1 | 10.7 | 8.3 | 6.1 |

Evidently , the degradation products of the dexamethasone palmitate injections increase as the pH grows; In the formulation of the 5% glycerol with pH 10, the degradation products decrease from 18.6% to 10.7%; when the glycerol reaches 10%, the products further decrease from 18.6% to 6.1%, significantly enhancing the formulation stability.

**Experiment 14 glycerol reduces the PFAT5 of fat emulsions in the plastic bottles, increasing the compatibility safety of the formulations.**

Clinical infusion of the emulsions should go through the plastic infusion tube (set); in addition, some containers of the fat emulsions are plastic, especially for the multi-chamber bags of total nutrition, the plastic is more common, e.g. Instant Three-ChamerParenteral Nutrition Product Clinomel from Guangzhou Baxter International Inc., therefore it is necessary to survey on the glass and plastic materials.

Similar to the Experiment 2 (20% fat emulsion injection, Sichuan Kelun Pharmaceutical Co.,Ltd.), The adding of glycerol (on the clean bench)to the make the glycerol (w/v) as follows: 3.0%, 5.0%, 10.0% and 15.0% (0.005% EDTA-2Na in Each for containing the microbe), packaged in the non-PVC bags and the emulsions are shaken (10rpm) for 12h, and the results are shown in table 31.

**Table 31 glycerol of different concentrations reduces the PFAT5 of the fat emulsions in the plastic bottles**

| | glycerol (w/v) | | | | |
|---|---|---|---|---|---|
| | 2.5% | 3.0% | 5.0% | 10.0% | 15.0% |
| PFAT(5) (0h) | 0.026% | 0.018% | 0.022% | 0.016% | 0.028% |
| PFAT(5) (12h) | 0.338% | 0.102% | 0.046% | 0.036% | 0.041% |

From the table, high concentration glycerol remarkably reduces the PFAT (5). Based on the standard USP<729>, requirements of PFAT(5), i.e. particles of more than 5um in size, less than 0.05% in quantity; when the glycerol is greater than or equal to 5%, the fat emulsion is qualified and resists the shaking destruction.

**Experiment 15 effect of freeze-thaw treatment on the glycerol of the formulations**

Glycerol has been used as the osmotic regulator for the emulsion, and the concentrations are less than 2.5%. the freezing points are different as the glycerol concentration varies, i.e. the 10%, 30%, 50%, 66.7%, 80%, 90% glycerol are frozen at -1.6°C, -9.5°C, -23.0°C,-46.5°C,-20.3°C and -1.6°C .from that , even the glycerol in the emulsion reaches 30%, its freezing point is only -9.5°C, far lower than the freeze-thaw temperatures and the ice crystal is bound to appear. And why adding 3%-10% glycerol (10% glycerol: -1.6°C) solve the issue? To explore how the glycerol enhances the freeze-thaw resistance of the emulsions/or glycerol enables the fat emulsion to tolerate the freeze-thaw, inventors measure the glycerol after the freeze-thaw cycles.

Glycerol Detection: HPLC-ELSD for measuring the glycerol in the M/L fat emulsions, HPLC conditions: Column, InterstiNH2 (250mm*4.6mm, 5um); Column Temperature, 30°C; Mobile phase, acetonitrile: water (90:10, V/V); speed, 1.0 mL·min$^{-1}$; Injection, 100$\mu$L; ELSD detector drift tube temperature, 40°C; gain value, 1.0; pressure, 1.6 Bar.

Experiment: The fat emulsions are purchased from the hospital, comprising medium-chain and long chain fat emulsion injection (Germany/B.Braun Medical (Suzhou)) Company) (LM-20-2.5,10% LCT, 10% MCT, 1.2% EPC, 2.5% glycerol) (package batch No.38112146, Product batch No.122538084), labeled as ML-20-2.5%, 20% fat emulsion injection (L-20, 20% LCT, 1.2% EPC, 2.2% Glycerol, Sichuan Kelun Pharmaceutical Co., Ltd.( Product Batch No.B13061903-2), labeled as L-20-2.2%), 30% fat emulsion injection (L30, 30% LCT, 1.2%EPC, 1.67%Glycerol, Xi'an Libang Pharmaceutical (13090211), labeled as L-30-1.67%), Structural fat emulsion injection (20%STG, 1.2% EPC, 2.2%glycerol, Wuxi SSPC (10GE2883), labeled as STG-20-2.2%), which undergo the freeze-thaw treatment and centrifuged at low temperature at 50000/min for 2h to separate the oil from the water. 100$\mu$l water layer is injected into the HPLC and the peak area of the glycerol is recorded to calculate the change of the glycerol before and after the freeze-thaw experiment as shown in table 32.

**Table 32 glycerol changes of the market-selling fat emulsion injections before and after the freeze-thaw experiments**

| Freeze-thaw cycle (s) | ML-20-2.5% | L-20-2.2% | L-30-1.67% | STG-20-2.2% |
|---|---|---|---|---|
| 1 | -2.81 | -3.19 | -5.46 | -7.17 |
| 2 | -1.59 | -2.36 | -3.93 | -13.72 |
| 3 | -3.88 | -5.20 | -10.43 | -32.38 |

From the results, the glycerol in the water phase falls and it enter into the emulsion layer, especially for the L-30-1.67%, the change is the largest, i.e. 30% glycerol at loss in the water phase after three cycles. In addition, the glycerol change is connected with the oil concentration based on the formula analysis. The oil of the same concentration (ML-20-2.5%, L-20-2.2% and STG-20-2.2%), glycerol loss of the structural fat emulsion is more than that of the long-chain fat oil (soy bean oil) and ML emulsions; when the soybean oil is the same (L-20-2.2% and L-30-1.67%), the glycerol loss of the 30% emulsion is less than that of 20% emulsion.

Inventors add glycerol into the ML-20-2.5% to make the concentration as follows: 3%, 5%, 10% and 15%, labeled as ML-20-3%, ML-20-5%, ML-20-10% and ML-20-15%. The same method is employed to calculate the glycerol change before and after the freezing and thawing as shown in table 33.

**Table 33 glycerol change of the fat emulsions during freeze-thaw experiments**

| Freeze-thaw cycle (s) | ML-20-3% | ML-20-5% | ML-20-10% | ML-20-15% |
|---|---|---|---|---|
| 1 | 0.36 | 4.22 | 7.64 | 8.23 |
| 2 | -0.55 | 3.21 | 12.53 | 11.06 |
| 3 | 1.03 | 5.72 | 14.28 | 12.40 |

Evidently, when the glycerol reaches 5% or more, three cycles will cause any glycerol loss, in contrast, a little in rise.

According to the inventor's hypothesis, when the glycerol is less than or equal to 2.5%, the fat emulsion will produce crystals during the freezing to concentrate the glycerol and spread to the emulsion surface to fill in the gaps; when the glycerol is more than 3%, the glycerol completely covers the emulsion surface, and the freezing crystal will reduce the glycerol in the water phase. In addition, inventors find it needs more glycerol when the oil concentration is higher, which opposes to the theory of the glycerol regulating the osmotic pressure (e.g. 10% fat emulsion, glycerol 2.5%; 30% fat emulsion, glycerol 1.67%). In summary, the inventors conclude the differential distribution of the glycerol in the water and emulsion surface, and the glycerol in the emulsion surface is higher than that in the water phase.

**Experiment 16 the making of 20% LCT fat emulsions of different glycerol concentrations and their freeze-thaw stability investigation**

1. The making of 20% LCT fat emulsions of different glycerolconcentrations

The formula of 20% LCT fat emulsions of different glycerolconcentrations is shown in Table 34.

**Table 34 formula of 20% LCT fat emulsions of different glycerolconcentrations**

| Ingredients | Glycerol (w/v) | | | | |
|---|---|---|---|---|---|
| | 2.2% | 3.0% | 6.0% | 10.0% | 15.0% |
| LCT | 20 g | 20 g | 20 g | 20 g | 20g |
| EPC | 1.2 g | 1.2 g | 1.2 g | 1.2 g | 1.2 g |
| Glycerol | 2.2 g | 3.0g | 6.0 g | 10.0 g | 15.0 g |
| Sodium Oleate | 0.02g | 0.02 g | 0.02 g | 0.02 g | 0.02 g |
| EDTA-2Na | 0.005 | 0.005 | 0.005 | 0.005 | 0.005 |
| Injection Water | Up to 100 mL | Up to 100 mL | Up to 100 mL | Up to 100 mL | Up to100mL |

The process is carried out according to the example 7.

2. The freeze-thaw stability investigation of 20% LCT fat emulsions of different glycerol concentrations

20% LCT fat emulsions of different glycerol concentrations are for the freeze-thaw experiment comprising the following steps: put some bottles of the emulsions in the -20°C for 48h, then 40 °C for 48h as a cycle, measure the particle size in each cycle, and the results are shown in table 35.

**Table 35 results of freeze-thaw stability of 20% LCT fat emulsions of different glycerolconcentrations**

| Freeze-thaw cycle (s) | Glycerol (w/v) | | | | | | | | | |
|---|---|---|---|---|---|---|---|---|---|---|
| | 2.2% | | 3.0% | | 6.0% | | 10.0% | | 15.0% | |
| | MD | CV | MD | CV | MD | CV | MD | CV | MD | CV |
| 0 | 296.6 | 0.560 | 296.6 | 0.560 | 299.5 | 0.449 | 293.6 | 0.402 | 299.9 | 0.415 |
| 1 | separation | -- | Oil floating | -- | 293.9 | 0.593 | 298.0 | 0.432 | 292.7 | 0.542 |
| 2 | separation | -- | Oil floating | -- | 300.2 | 0.516 | 301.5 | 0.499 | 301.0 | 0.422 |
| 3 | separation | -- | separation | -- | 300.9 | 0.515 | 301.8 | 0.465 | 300.9 | 0.419 |

From the results, when the glycerol is 2.2% and 3.0%, the 20% LCT fat emulsions cannot tolerate the freeze-thaw experiment, i.e. not freeze-thaw resistant. when the glycerol is greater than or equal to the 1/3 of the oil (w/w), i.e. that glycerol is more than or equal to 6.7%, the 20% LCT fat emulsion can tolerate the experiment and the freeze-thaw resistant 20% LCT fat emulsion is obtained.

**Experiment 17 the making of 20% MCT fat emulsions of different glycerolconcentrations and their freeze-thaw stability investigation**

Inventors replace the LCT in the Experiment 16 with MCT, and make 20% MCT fat emulsion of different glycerolconcentrations and investigate the freeze-thaw stability as shown in table 36.

**Table 36 results of freeze-thaw stability of 20% MCT fat emulsions of different glycerolconcentrations**

| Freeze-thaw cycle (s) | Glycerol ( w/v ) | | | | | | | | | |
|---|---|---|---|---|---|---|---|---|---|---|
| | 2.2% | | 3.0% | | 6.0% | | 10.0% | | 15.0% | |
| | MD | CV | MD | CV | MD | CV | MD | CV | MD | CV |
| 0 | 297.6 | 0.460 | 291.6 | 0.560 | 286.5 | 0.449 | 280.6 | 0.402 | 269.6 | 0.315 |
| 1 | separation | -- | 1543.6 | 0.863 | 283.9 | 0.493 | 288.0 | 0.432 | 272.7 | 0.342 |
| 2 | separation | -- | Oil floating | -- | 290.2 | 0.416 | 281.5 | 0.499 | 271.0 | 0.422 |
| 3 | separation | -- | separation | -- | 291.9 | 0.415 | 287.8 | 0.465 | 270.9 | 0.419 |

From the results, when the glycerol is 2.2% and 3.0%, the 20% MCT fat emulsions cannot tolerate the freeze-thaw experiment, i.e. not freeze-thaw resistant. When the glycerol is greater than or equal to the 1/3 of the oil (w/w), i.e. that glycerol is more than or equal to 6.0%, the 20% LCT fat emulsion can tolerate the experiment and the freeze-thaw resistant 20% MCT fat emulsion is obtained.

**Experiment 18 the making of 20% structural fat emulsions of different glycerolconcentrations and their freeze-thaw stability investigation**

Inventors replace the LCT in the example 16 with the structural oil of the medium-chain and long-chain fatty acid, and make 20% structuralfat emulsion of different glycerolconcentrations and investigate the freeze-thaw stability as shown in table 37.

**Table 37 results of freeze-thaw stability of 20% structural fat emulsions of different glycerolconcentrations**

| Freeze-thaw cycle (s) | Glycerol (w/v) | | | | | | | | | |
|---|---|---|---|---|---|---|---|---|---|---|
| | 2.2% | | 5.0% | | 6.7% | | 10.0% | | 15.0% | |
| | MD | CV | MD | CV | MD | CV | MD | CV | MD | CV |
| 0 | 279.5 | 0.319 | 276.2 | 0.328 | 247.8 | 0.352 | 251.2 | 0.381 | 245.0 | 0.309 |
| 1 | separation | -- | Oil floating | -- | 248.1 | 0.445 | 253.4 | 0.420 | 245.9 | 0.387 |
| 2 | separation | -- | separation | -- | 252.5 | 0.398 | 259.1 | 0.350 | 250.0 | 0.267 |
| 3 | separation | -- | separation | -- | 250.4 | 0.446 | 258.0 | 0.230 | 251.2 | 0.426 |

From the results, when the glycerol is 2.2% and 5.0%, the 20% structural fat emulsions cannot tolerate the freeze-thaw experiment,i.e. not freeze-thaw resistant. When the glycerol is greater than or equal to the 1/3 of the oil (w/w), i.e. that glycerol is more than or equal to 6.7%, the 20% structural fat emulsion can tolerate the experiment and the freeze-thaw resistant 20% structural fat emulsion is obtained.

**Experiment 19 the making of 20% medium-chain and long-chain fat emulsions of different glycerolconcentrations and their freeze-thaw stability investigation**

Inventors replace the LCT in the example 16 with 20% LCT and 10% MCT, and make 20% M/L fat emulsion of different glycerolconcentrations and investigate the freeze-thaw stability as shown in table 38.

**Table 38 results of freeze-thaw stability of 20% M/L fat emulsions of different glycerolconcentrations**

| Freeze-thaw cycle (s) | Glycerol (w/v) | | | | | | | | | |
|---|---|---|---|---|---|---|---|---|---|---|
| | 2.5% | | 5.0% | | 7.5% | | 10.0% | | 15.0% | |
| | MD | CV | MD | CV | MD | CV | MD | CV | MD | CV |
| 0 | 284.4 | 0.550 | 286.7 | 0.474 | 284.5 | 0.519 | 294.4 | 0.542 | 293.6 | 0.549 |
| 1 | 1563.4 | 0.875 | 337.6 | 0.471 | 273.2 | 0.560 | 295.9 | 0.453 | 281.5 | 0.517 |
| 2 | separation | -- | Oil floating | -- | 289.6 | 0.432 | 303.8 | 0.439 | 297.6 | 0.391 |
| 3 | separation | -- | separation | -- | 290.0 | 0.426 | 317.4 | 0.386 | 298.0 | 0.392 |

From the results, when the glycerol is 2.5% and 5.0%, the 20% M/L fat emulsions cannot tolerate the freeze-thaw experiment, i.e. not freeze-thaw resistant. When the glycerol is greater than or equal to the 1/3 of the oil (w/w), i.e. that glycerol is more than or equal to 6.7%, the 20% M/L fat emulsion can tolerate the experiment and the freeze-thaw resistant 20% M/L fat emulsion is obtained.

Inject the mouse tail veins with 0.2ml/10g one-cycle fat emulsion and record the death in 24h. the result is that the mortalities of the 2.5% and 5% groups are 80% and 60% respectively, while no death is found in the glycerol 7.5%, 10% and 15%, which proves the glycerol of eliminating the toxicity from the freezing-thawing.

**Experiment 20 the making of 30% LCT fat emulsions of different**

**glycerolconcentrations and their freeze-thaw stability investigation**

1. The making of 30% LCT fat emulsions of different glycerolconcentrations

The formula of 30% LCT fat emulsions of different glycerolconcentrations is shown in table 39.

**Table 39 formula of 30% LCT fat emulsions of different glycerolconcentrations**

| Ingredients | Glycerol (w/v) | | | | |
|---|---|---|---|---|---|
| | 1.67% | 8% | 10% | 15% | 20% |
| EPC | 1.8 g | 1.8 g | 1.8 g | 1.8 g | 1.8g |
| LCT | 30 g | 30 g | 30 g | 30 g | 30g |
| Glycerol | 1.67 g | 8g | 10 g | 15 g | 20 g |
| Sodium Oleate | 0.02g | 0.02 g | 0.02 g | 0.02 g | 0.02 g |
| Sodium Hydroxide | proper | proper | proper | proper | proper |
| Injection Water | Up to 100 mL | Up to 100 mL | Up to 100 mL | Up to 100 mL | Up to 100mL |

The process is carried out according to the example 7.

2. The freeze-thaw stability investigation of 30% LCT fat emulsions of different glycerolconcentrations

30% LCT fat emulsions of different glycerolconcentrations are for the freeze-thaw experimentscomprising the following steps: put some bottles of the emulsions in the -20 °C for 48h, then 40 °C for 48h as a cycle, measure the particle size in each cycle, and the result is shown in table 40.

**Table 40 results of freeze-thaw stability of 30% LCT fat emulsions of different glycerolconcentrations**

| Freeze-thaw cycle (s) | Glycerol (w/v) | | | | | | | | | |
|---|---|---|---|---|---|---|---|---|---|---|
| | 1.67% | | 5% | | 10% | | 15% | | 20% | |
| | MD | CV | MD | CV | MD | CV | MD | CV | MD | CV |
| 0 | 358.8 | 0.589 | 331.5 | 0.575 | 330.8 | 0.455 | 322.6 | 0.472 | 311.1 | 0.381 |
| 1 | separation | -- | 1579.4 | 0.768 | 338.0 | 0.471 | 324.9 | 0.407 | 317.0 | 0.443 |
| 2 | separation | -- | Oil floating | -- | 334.9 | 0.496 | 329.4 | 0.435 | 317.6 | 0.474 |
| 3 | separation | -- | separation | -- | 337.1 | 0.483 | 322.5 | 0.494 | 310.0 | 0.358 |

From the results, when the glycerol is 1.67% and 8%, the 30% LCT fat emulsions cannot tolerate the freeze-thaw experiment, i.e. not freeze-thaw resistant. When the glycerol is greater than or equal to 10%, the 30% LCT fat emulsion can tolerate the experiments and the freeze-thaw reistant 30% LCT fat emulsion is obtained.

**Experiment 21 the making of 10% seabuckthorn seed oil fat emulsions of different glycerolconcentrations and their freeze-thaw stability investigation**

Inventors make 30% MCT fat emulsions of different glycerol concentrations according to the example 8 and investigate their freeze-thaw stability as shown in table 41.

**Table 40 results of freeze-thaw stability of 10% seabuckthorn seed oil fat emulsions of different glycerolconcentrations**

| Freeze-thaw cycle (s) | Glycerol (w/v) | | | | | | | | | |
|---|---|---|---|---|---|---|---|---|---|---|
| | 1.67% | | 8% | | 10% | | 15% | | 20% | |
| | MD | CV | MD | CV | MD | CV | MD | CV | MD | CV |
| 0 | 341.1 | 0.581 | 320.5 | 0.581 | 322.5 | 0.454 | 310.8 | 0.449 | 300.0 | 0.412 |
| 1 | separation | -- | 1423.6 | 0.786 | 327.9 | 0.506 | 309.1 | 0.332 | 301.2 | 0.456 |
| 2 | separation | -- | separation | -- | 322.2 | 0.407 | 314.7 | 0.317 | 304.5 | 0.397 |
| 3 | separation | -- | separation | -- | 329.1 | 0.435 | 315.5 | 0.480 | 308.5 | 0.371 |

From the results, when the glycerol is 1.67% and 8%, the 30% MCT fat emulsions cannot tolerate the freeze-thaw experiment, i.e. not freeze-thaw resistant. When the glycerol is greater than or equal to 10%, the 30% MCT fat emulsion can tolerate the experiment and the freeze-thaw resistant 30% MCT fat emulsion is obtained.

**Experiment 22 effect of NaCl concentrations on the freeze-thaw stability of the medium-chain and long-chain fat emulsions of different glycerol concentrations**

Proper amount of the market-selling 20% M/L fat emulsion injections (ML-20) is divided into two, one is diluted by 1 fold by 0.9% NaCL, the other is diluted by 1fold by 1.8% NaCl, both of which are filled into the 3ml penicillin bottle with 2ml in each and 14 bottles in total; the adding of glycerol into the injection to render the glycerol concentration as follows: 1.25%, 2.5%, 3.6%, 5%, 7.5%, 10% and 20%. Two bottles are for each concentration, followed by magnetic stirring for 20min, then the emulsions are for the freeze-thaw stability experiments, and the results are shown in tables 42 and 43.

**Table 42 effect of 0.9% NaCl on the freeze-thaw stability of the market-selling 20% medium-chain and long-chain fat emulsions of different glycerol concentrations**

| | 2.5% | | 3.0% | | 5% | | 7.5% | | 10% | | 20% | |
|---|---|---|---|---|---|---|---|---|---|---|---|---|
| | MD | CV | MD | CV | MD | CV | MD | CV | MD | CV | MD | CV |
| 0 | 295.4 | 0.417 | 295.0 | 0.348 | 297.7 | 0.397 | 295.6 | 0.424 | 287.7 | 0.376 | 302.7 | 0.392 |
| 1 | 361.0 | 0.448 | 290.1 | 0.343 | 289.5 | 0.318 | 289.7 | 0.427 | 289.7 | 0.345 | 286.4 | 0.373 |
| 2 | Oil floating | - | 301.0 | 0.452 | 294.6 | 0.456 | 294.8 | 0.435 | 299.5 | 0.327 | 304.1 | 0.442 |
| 3 | Oil floating | - | 311.4 | 0.476 | 321.7 | 0.498 | 311.6 | 0.402 | 298.8 | 0.360 | 311.1 | 0.404 |

**Table 43 effect of 1.8% NaCl on the freeze-thaw stability of the market-selling 20% medium-chain and long-chain fat emulsions of different glycerol concentrations**

| | 2.5% | | 3.0% | | 5% | | 7.5% | | 10% | | 20% | |
|---|---|---|---|---|---|---|---|---|---|---|---|---|
| | MD | CV | MD | CV | MD | CV | MD | CV | MD | CV | MD | CV |
| 0 | 292.9 | 0.423 | 290.4 | 0.394 | 297.2 | 0.452 | 304.0 | 0.428 | 294.5 | 0.363 | 292.2 | 0.304 |
| 1 | 386.8 | 0.301 | 293.7 | 0.339 | 299.0 | 0.387 | 290.9 | 0.361 | 286.6 | 0.302 | 291.0 | 0.328 |
| 2 | Oil floating | - | 286.9 | 0.374 | 299.8 | 0.421 | 297.1 | 0.459 | 293.6 | 0.479 | 290.3 | 0.463 |
| 3 | Oil floating | - | 321.2 | 0.494 | 313.6 | 0.488 | 308.2 | 0.380 | 318.0 | 0.348 | 310.9 | 0.483 |

Evidently, the glycerol increase can improve the capability to resist the electrolyte and enhance the compatibility stability of the fat emulsions with the NaCl injection.

**Embodiments**

**Example 1 the making of the freeze-thaw resistant tanshinone IIA fat emulsionsand their freeze-thaw stability investigation**

1. The making of the freeze-thaw resistant tanshinone IIA fat emulsions

The formula of the freeze-thaw resistant tanshinone IIA fat emulsions: tanshinone IIA 0.1g; MCT 10g; LCT 10g; EPC 1.4g; glycerol 8g; sodium oleate 0.02g; disodium hydrogen phosphate proper concentration; injection water up to 100ml.

Process: mix the tanshinone IIA, EPC, LCT and MCT to constitute the oil phase; mix the sodium oleate and 85% (v/v) injection water to constitute the water phase; both are heated to 60°C. When the materials of the oil completely dissolve, the water phase is added into the oil phase slowly assisted by the magnetic stirring. Continue the stirring for 20 min and obtain the pre-emulsion. The pre-emulsion is homogenized five times by a microfluider, diluted by the sterile injection water up to 100ml and filtered by the 0.45 $\mu$m microfiler. The pH of the solution is adjusted to 8.2 by 10mM disodium hydrogen phosphate. The solution fills the 10ml penicillin bottle to full scale and the bottles are filled with nitrogen and plugged and covered with aluminum caps; the bottles are sterilized in 121 °C for 10 min, followed by cold water sprinkling the emulsion to RT. The emulsion is done.

2. The investigation of the freeze-thaw stability of the freeze-thaw resistant tanshinone IIA fat emulsions

Testing steps: put the tanshinone IIA emulsions in the -20°C for 48h, then 40 °C for 48h as a cycle, measure the particle size in each cycle, and the result is shown in table 44.

**Table 44 results of the freeze-thaw stability of the freeze-thaw resistant tanshinone IIA fat emulsions**

| Freeze-thaw cycle (s) | MD | CV |
|---|---|---|
| 0 | 234.8 | 0.325 |
| 1 | 232.6 | 0.357 |
| 2 | 238.9 | 0.421 |
| 3 | 240.6 | 0.411 |

From the results, the tanshinone IIA fat emulsions tolerate the cycles, i.e. the freeze-thaw resistant tanshinone IIA fat emulsions are obtained.

**Example 2 the making of the freeze-thaw resistant Bruceajavanicaseed oil emulsions and their freeze-thaw stability investigation**

1. The making of the freeze-thaw resistant Bruceajavanicaseed oil emulsions

Formula: Bruceajavanica seed oil 5g; coix seed oil 5g; EPC 1.2g; glycerol 10g; sodium citrate proper concentration; injection water up to 100ml.

Process: mix the Bruceajavanica seed oil,coix seed oil and EPC to constitute the oil phase; mix the sodium oleate and 85% (v/v) injection water to constitute the water phase; both are heated to 60 °C . When the materials of the oil completely dissolve, the water phase is added into the oil phase slowly assisted by the magnetic stirring. Continue the stirring for 20 min and obtain the pre-emulsion. The pre-emulsion is homogenized five times by a microfluider, diluted by the sterile injection water up to 100ml and filtered by the 0.45$\mu$m microfiler. The pH of the solution is adjusted to 8.2 by 10mM sodium citrate. The solution fills the 10ml penicillin bottle to full scale and the bottles are filled with nitrogen and plugged and covered with aluminum caps; the bottles are sterilized in 121 °C for 10 min, followed by cold water sprinkling the emulsion to RT. The emulsion is done.

2. The investigation of the freeze-thaw stability of the freeze-thaw resistant Bruceajavanica seed oil emulsions

Testing steps: put the Bruceajavanica seed oil emulsions in the -20°C for 12h, then RT for 3h as a cycle, measure the particle size in each cycle, and the result is shown in table 45.

Table 45 results of the freeze-thaw stability of the freeze-thaw resistant Bruceajavanicaseed oil emulsions

| Freeze-thaw cycle (s) | MD | CV |
|---|---|---|
| 0 | 237.2 | 0.335 |
| 1 | 241.6 | 0.317 |
| 2 | 238.4 | 0.325 |
| 3 | 243.1 | 0.316 |

From the result, the Bruceajavanicaseed oil emulsions tolerate the cycles.

**Example 3 the making of the freeze-thaw resistant curcuminfat emulsions and their freeze-thaw stability investigation**

1. The making of the freeze-thaw resistant curcumin fat emulsions

Formula: curcumin 0.5g; SPC 1.2g; LCT 15g; glycerol 20g; sodium hydroxide proper; injection water up to 100ml.
Process: similar to the process of the example 1.

2. The investigation of the freeze-thaw stability of the freeze-thaw resistant curcumin fat emulsions

Testing steps: put the curcumin fat emulsions in the -20°C for 48h, then 40 °C for 48h as a cycle, measure the particle size in each cycle, and the result is shown in table 46.

Table 46 results of freeze-thaw stability of the freeze-thaw resistant curcumin fat emulsions

| Freeze-thaw cycle (s) | MD | CV |
|---|---|---|
| 0 | 172.4 | 0.363 |
| 1 | 175.8 | 0.357 |
| 2 | 178.9 | 0.321 |
| 3 | 171.6 | 0.331 |

From the results, the curcumin fat emulsions tolerate the cycles, i.e. the freeze-thaw resistant Itraconazole fat emulsions are obtained.

**Example 4 the making of the freeze-thaw resistant prostaglandin E1 fat emulsions and their freeze-thaw stability investigation**

1. The making of the freeze-thaw resistant prostaglandin E 1 fat emulsions

Formula: prostaglandin E1 1mg; DOPC 1.2g; LCT 10g; glycerol 15g; sodium hydroxide proper; injection water up to 100ml.
Process: similar to the process of the example 1.

2. The investigation of the freeze-thaw stability of the freeze-thaw resistant prostaglandin E 1 fat emulsions

Testing steps: put the prostaglandin E1 fat emulsions in the -20°C for 48h, then 40 °C for 48h as a cycle, measure the particle size in each cycle, and the result is shown in table 47.

**Table 47 results of the freeze-thaw stability of the freeze-thaw resistant prostaglandin E1 fat emulsions**

| Freeze-thaw cycle (s) | MD | CV |
|---|---|---|
| 0 | 137.6 | 0.323 |
| 1 | 135.1 | 0.341 |
| 2 | 138.4 | 0.357 |
| 3 | 141.5 | 0.363 |

From the results, the prostaglandin E1 fat emulsions tolerate the cycles, i.e. the freeze-thaw resistant prostaglandin E1 fat emulsions are obtained.

More importantly, inventors find, when adding citric acid/ sodium citrate buffer into the emulsion to reach the end concentration 0.5mM and adjusting the pH to 5.0, the addition of glycerol will increase the viscosity of the water phase and stability, enlarging the pH range, e.g. 4.5-6.0.

Analogs to the prostaglandin, like limaprost, will have the same results through the said making process.

**Example 5 the making of the freeze-thaw resistant compound propofol fat emulsions and their freeze-thaw stability investigation**

1. The making of the freeze-thaw resistant compound propofol fat emulsions

Formula : propofol 1g; lidocaine hydrochloride 100mg; SPC 1.4g; LCT 10g; glycerol 10g; sodium hydroxide proper; injection water up to 100ml.

Process: similar to the process of the example 1.

2. The investigation of the freeze-thaw stability of the freeze-thaw resistant compound propofol fat emulsions

Testing steps: put the compound propofol fat emulsions in the -20°C for 48h, then 40 °C for 48h as a cycle, measure the particle size in each cycle, and the result is shown in table 48.

**Table 48 results of the freeze-thaw stability of the freeze-thaw resistant compound propofol fat emulsions**

| Freeze-thaw cycle (s) | MD | CV |
|---|---|---|
| 0 | 164.6 | 0.331 |
| 1 | 166.1 | 0.337 |
| 2 | 163.4 | 0.328 |
| 3 | 168.5 | 0.349 |

From the results, the compound propofol fat emulsions tolerate the cycles, i.e. the freeze-thaw resistant compound propofol fat emulsions are obtained.

**Example 6 glycerol resists the freeze-thaw destruction of the propofol fat emulsions**

The making of propofol fat emulsions: based on DIPRIVAN® (disoprofol 10mg/ml, soybean oil 100mg/ml, glycerol 22.5mg/ml, EPC 12mg/ml and EDTA-2Na( 0.005%) pH of 7-8.5), formula is shown in table 49.

**Table 49 formula of the freeze-thaw resistant compound propofol fat emulsions**

| Ingredients | Contents |
|---|---|
| Propofol | 2g |
| LCT | 5g |
| MCT | 5g |
| EPC | 1.2 g |

(continued)

| Ingredients | Contents |
|---|---|
| Glycerol | 3.0g (5.0g and10.0g) |
| EDTA-2Na | 0.005g |
| Injection Water | Up to 100 mL |

Process: similar to the process of the example 10.

The result shows: all emulsions tolerate three cycles, and 5% and 10% glycerol formula can tolerate five cycles.

Any analogs, such as anisole, asarone, elemene, will have the similar results according to the similar making process.

**Example 7 glycerol reduces the irritation of the disoprofol (propofol) fat emulsions.** 1. The making of the disoprofol fat emulsions

Based on DIPRIVAN®formula (disoprofol 10 mg/mL, soybean oil 100 mg/mL, glycerol 22.5 mg/mL, EPC12 mg/mL and EDTA-2Na (0.005%) pH of 7-8.5), inventors make emulsions of different glycerol concentrations (2.25%, 5.0%, 10.0%, 15.0%, 30.0% and 50.0%, labeled as 1% P-10%L-2.25, 1%P-10%L-5.0, 1%P-10%L-10.0, 1%P-10%L-15.0, 1%P-10%L-30.0 and 1%P-10%L-50.0, the formula is shown in table 50.

**Table 50 basic formula of the propofol fat emulsions**

| Ingredients | Contents |
|---|---|
| Propofol | 1g |
| LCT | 10g |
| EPC | 1.2 g |
| Glycerol | 2.25g (5.0g, 10.0g, 15.0g, 30.0g and 50.0g) |
| EDTA-2Na | 0.005g |
| Injection Water | Up to 100 mL |

Process: based on the example 1. When the glycerol is 30% or 50%, oil floats. No further work is carried out.

The particle sizes of the emulsions are shown in table 51.

**Table 51 particle sizes of the propofol fat emulsions**

| 1%P-10%L-2.25 (2.25%) | | 1%P-10%L-5 (5%) | | 1%P-10%L-10(10%) | | 1%P-10%L-15 (15%) | |
|---|---|---|---|---|---|---|---|
| MD | CV | MD | CV | MD | CV | MD | CV |
| 263.2 | 0.406 | 259.6 | 0.411 | 266.0 | 0.355 | 261.3 | 0.366 |

Evidently, the increase in glycerol will decrease the CV, i.e. narrower distribution of the particle sizes, and enhanced uniformity of the emulsion sizes.

2. The irritation of the propofol fat emulsions

30 SD rats are divided into 6 groups with 5 in each, which are anaesthetized by ether and laid on the backs on the rat platform. The emulsions are administered in the femoral arteryof the right hind legs with the dose of 0.5ml, recorded the areas by the electromyography. Compared with the electromyographic area of 1%P-10%L-2.25, the relative area ratios of 1%P-10%L-50, 1%P-10%L-10.0 and 1%P-10%L-15.0 are calculated which turn out 86.2%, 63.5% and 51.2% respectively, i.e. the high-glycerol groups reduce nearly half of the irritation caused by the propofol.

The possible mechanism of the glycerol reducing the irritation of the propofol is the interaction of the glycerol with the propofol through hydrogen bondssurrounding the propofol; meanwhile the glycerol covers the emulsion surface to reduce the contact of the propofol with the emulsions and the vessel walls, or the good compatibility of the propofol with the glycerol by combinations of hydroxyls and skeletons, or because the propofol is less soluble in water, the formulation aggregates to enlarge the volume and enhance the interaction with the vessel walls.

The penicillin bottle and pre-filled syringe are used to contain the propofol emulsion, shaken in 10rpm for 1 week. The PFAT 5 is calculated as shown in Table 52.

**Table 52 PFAT5 of the macroparticles of the propofol fat emulsions**

| 1%P-10%L-2.25 (2.25%) | | 1%P-10%L-5 (5%) | | 1%P-10%L-10(10%) | | 1%P-10%L-15 (15%) | |
|---|---|---|---|---|---|---|---|
| Penicillin Bottle | Pre-filled Syringe | Penicillin Bottle | Pre-filled Syringe | Penicillin Bottle | Pre-filled Syringe | Penicillin Bottle | Pre-filled Syringe |
| 0.16 | 0.35 | 0.08 | 0.11 | 0.03 | 0.05 | 0.03 | 0.05 |

Evidently the increase of the glycerol can increase the physical stability of the emulsions in the pre-filled syringes and reduce the macroparticles.

**Example 8 the making of the freeze-thaw resistant ketoprofen isopropyl ester fat emulsions and their freeze-thaw stability investigation**

1. The making of the freeze-thaw resistant ketoprofen isopropyl ester fat emulsions

**Table 53 formula of the freeze-thaw resistant ketoprofen isopropyl ester fat emulsions**

| Ingredients | Contents |
|---|---|
| Ketoprofen isopropyl ester | 1.16 g |
| EPC | 1.4 g |
| LCT | 10 g |
| Glycerol | 5 g |
| Sodium Oleate | 0.01 g |
| Sodium Hydroxide | proper |
| Injection Water | Up to 100 mL |

Process: similar to the process of the example 1.

2. The investigation of the freeze-thaw stability of the freeze-thaw resistant ketoprofen isopropyl ester fat emulsions

Testing steps: put the ketoprofen isopropyl ester fat emulsions in the -20°C for 48h, then 40 °C for 48h as a cycle, measure the particle size in each cycle, and the result is shown in table 54.

**Table 54 results of the freeze-thaw stability of the freeze-thaw resistant ketoprofen isopropyl ester fat emulsions**

| Freeze-thaw cycle (s) | MD | CV |
|---|---|---|
| 0 | 176.9 | 0.411 |
| 1 | 177.4 | 0.367 |
| 2 | 182.9 | 0.378 |
| 3 | 180.4 | 0.352 |

From the results, the ketoprofen isopropyl ester fat emulsions tolerate the cycles, i.e. the freeze-thaw resistant ketoprofen isopropyl ester fat emulsions are obtained.

**Example 9 the making of the freeze-thaw resistant malotilate fat emulsions and their freeze-thaw stability investigation**

1. The making of the freeze-thaw resistant malotilate fat emulsions

Table 55 formula of the freeze-thaw resistant malotilate fat emulsions

| Ingredients | Contents |
|---|---|
| Malotilate | 0.2 g |
| EPC | 1.4 g |
| STC | 10 g |
| Glycerol | 10 g |
| Oleic Acid | 0.05 g |
| Sodium Hydroxide | proper |
| Injection Water | Up to 100 mL |

Process: similar to the process of the example 1.

2. The investigation of the freeze-thaw stability of the freeze-thaw resistant malotilate fat emulsions

Testing steps: put the malotilate fat emulsions in the -20 °C for 48h, then 40 °C for 48h as a cycle, measure the particle size in each cycle, and the result is shown in table 56.

Table 56 results of the freeze-thaw stability of the freeze-thaw resistant malotilate fat emulsions

| Freeze-thaw cycle (s) | MD | CV |
|---|---|---|
| 0 | 190.9 | 0.331 |
| 1 | 193.1 | 0.325 |
| 2 | 195.2 | 0.326 |
| 3 | 189.7 | 0.354 |

From the results, the malotilate fat emulsions tolerate the cycles, i.e. the freeze-thaw resistant malotilate fat emulsions are obtained.

**Example 10 the making of the freeze-thaw resistant cardy oil/EPA fat emulsions and their freeze-thaw stability investigation**

1. The making of the freeze-thaw resistant cardy oil/EPA fat emulsions

Table 57 formula of the freeze-thaw resistant cardy oil/EPA fat emulsions

| Ingredients | Contents |
|---|---|
| Cardy oil | 10.0 g |
| Tea oil | 10.0 g |
| EPA | 1g |
| EPC | 1.2 g |
| Glycerol | 7.5 g |

(continued)

| Ingredients | Contents |
|---|---|
| Oleic Acid | 0.1 g |
| Sodium Hydroxide | proper |
| Injection Water | Up to 100 mL |

Process: similar to the process of the example 1.

2. The investigation of the freeze-thaw stability of the freeze-thaw resistant cardy oil/EPA fat emulsions

Testing steps: put the cardy oil/EPA fat emulsions in the -20 °C for 48h, then 40 °C for 48h as a cycle, measure the particle size in each cycle, and the result is shown in table 58.

**Table 58 results of the freeze-thaw stability of the freeze-thaw resistant cardy oil/EPA fat emulsions**

| Freeze-thaw cycle (s) | MD | CV |
|---|---|---|
| 0 | 140.9 | 0.331 |
| 1 | 143.1 | 0.325 |
| 2 | 145.2 | 0.326 |
| 3 | 149.7 | 0.354 |

From the results, the cardy oil/EPA fat emulsions tolerate the cycles, i.e. the freeze-thaw resistant cardy oil/EPA fat emulsions are obtained.

**Example 11 the making of the low-concentration CoQ10 fat emulsions and their freeze-thaw stability investigation**

1. The making of the freeze-thaw resistant CoQ10 fat emulsions

The low-concentration CoQ 10 fat emulsions are designed based on the market-selling Q injection (2.5mg/ml) and the formula is shown in Table 59.

**Table 59 formula of the freeze-thaw resistant CoQ10fat emulsions**

| Ingredients | Contents |
|---|---|
| $CoQ_{10}$ | 0.25g |
| SPC | 0.5 g |
| LCT | 2.5 g |
| MCT | 2.5 g |
| Glycerol | 3g |
| Oleic Acid | 0.02 g |
| Sodium Hydroxide | proper |
| Injection Water | Up to 100 mL |

Process: similar to the process of the example 1.

1. The investigation of the freeze-thaw stability of the freeze-thaw resistant CoQ10 fat emulsions

Testing steps: put the$CoQ_{10}$ fat emulsions in the -20°C for 48h, then 40 °C for 48h as a cycle, measure the particle size in each cycle, and the result is shown in table 60.

Table 60 results of the freeze-thaw stability of the freeze-thaw resistantCoQ$_{10}$ fat emulsions

| Freeze-thaw cycle (s) | MD | CV |
|---|---|---|
| 0 | 171.2 | 0.351 |
| 1 | 172.5 | 0.342 |
| 2 | 175.2 | 0.341 |
| 3 | 172.6 | 0.327 |

From the results, the CoQ$_{10}$ fat emulsions tolerate the cycles, i.e. the freeze-thaw resistantCoQ$_{10}$ fat emulsions are obtained.

**Example 12 the making of the high drug-loading CoQ10 fat emulsions and their freeze-thaw stability investigation**

1. The making of the high drug-loading CoQ$_{10}$fat emulsions

Table 61 formula of the freeze-thaw resistant and high drug-loading CoQ10fat emulsions

| Ingredients | Contents |
|---|---|
| CoQ$_{10}$ | 2.0 g |
| E80 | 2.0 g |
| LCT | 15 g |
| MCT | 15 g |
| Glycerol | 10 g |
| Sodium Hydroxide | proper |
| Injection Water | Up to 100 mL |

Process: similar to the process of the example 1.

2. The investigation of the freeze-thaw stability of the freeze-thaw resistant and high drug-loading CoQ$_{10}$ fat emulsions

Testing steps: put the high drug-loadingCoQ$_{10}$ fat emulsions in the -20°C for 48h, then 40 °C for 48h as a cycle, measure the particle size in each cycle, and the result is shown in table 62.

Table 62 results of the freeze-thaw stability of the freeze-thaw resistant and high drug-loadingCoQ10 fat emulsions

| Freeze-thaw cycle (s) | MD | CV |
|---|---|---|
| 0 | 361.2 | 0.351 |
| 1 | 367.7 | 0.353 |
| 2 | 365.9 | 0.327 |
| 3 | 362.8 | 0.349 |

From the results, the high drug-loading CoQ$_{10}$ fat emulsions tolerate the cycles, i.e. the freeze-thaw resistant high drug-loading CoQ$_{10}$ fat emulsions are obtained.

**Example 13 the making of the freeze-thaw resistant garlic oil fat emulsions and their freeze-thaw stability investigation**

1. The making of the freeze-thaw resistant garlic oil fat emulsions

Formula: garlic oil 0.5g; EPC 1.3g; LCT 15g; glycerol 7.5g; oleic acid 0.1g; sodium hydroxide proper concentration; injection water up to 100ml

Process: similar to the process of the example 1.

2. The investigation of the freeze-thaw stability of the freeze-thaw resistant garlic oil fat emulsions

Testing steps: put the garlic oil fat emulsions in the -20 °C for 48h, then 40 °C for 48h as a cycle, measure the particle size in each cycle, and the result is shown in table 63.

**Table 63 results of the freeze-thaw stability of the freeze-thaw resistantgarlic oil fat emulsions**

| Freeze-thaw cycle (s) | MD | CV |
|---|---|---|
| 0 | 152.4 | 0.334 |
| 1 | 157.1 | 0.318 |
| 2 | 159.3 | 0.324 |
| 3 | 155.4 | 0.319 |

From the results, the garlic oil fat emulsions tolerate the cycles, i.e. the freeze-thaw resistant garlic oil fat emulsions are obtained.

**Example 14 the making of the freeze-thaw resistant vitamin K1 fat emulsions and their freeze-thaw stability investigation**

1. The making of the freeze-thaw resistant vitamin K1 fat emulsions

**Table 64 formula of the freeze-thaw resistantvitamin K1 fat emulsions**

| Ingredients | Contents |
|---|---|
| Vitamin K1 | 0.2 g |
| SPC | 1.2 g |
| Vitamin E | 0.1 g |
| Olive Oil | 10 g |
| Glycerol | 7.5 g |
| EDTACa Na | 0.001 |
| Sodium Hydroxide | proper |
| Injection Water | Up to 100 mL |

Process: similar to the process of the example 1.

2. The investigation of the freeze-thaw stability of the freeze-thaw resistant vitamin K1 fat emulsions

Testing steps: put the vitamin K1 fat emulsions in the -20 °C for 48h, then 40 °C for 48h as a cycle, measure the particle size, and the result is shown in table 60.

**Table 65 results of the freeze-thaw stability of the freeze-thaw resistant vitamin K1 fat emulsions**

| Freeze-thaw cycle (s) | MD | CV |
|---|---|---|
| 0 | 235.4 | 0.324 |
| 1 | 237.1 | 0.338 |
| 2 | 236.3 | 0.354 |
| 3 | 255.8 | 0.349 |

From the results, the vitamin K1 fat emulsions tolerate the cycles, i.e. the freeze-thaw resistant vitamin K1fat emulsions are obtained.

**Example 15 the making of the freeze-thaw resistant cucurbitacin E fat emulsions and their freeze-thaw stability investigation**

1. The making of the freeze-thaw resistant cucurbitacin E fat emulsions

**Table 66 formula of the freeze-thaw resistant cucurbitacin E fat emulsions**

| Ingredients | Contents |
|---|---|
| CucurbitacinE | 0.005 g |
| EPC | 1.2 g |
| CoconutOil | 20 g |
| Glycerol | 30 g |
| Sodium Oleate | 0.03 g |
| Injection Water | Up to 100 mL |

Process: similar to the process of the example 1.

2. The investigation of the freeze-thaw stability of the freeze-thaw resistant cucurbitacin E fat emulsions

Testing steps: put thecucurbitacin Efat emulsions in the -20°C for 48h, then 40 °C for 48h as a cycle, measure the particle size in each cycle, 3 cycles. The result shows the cucurbitacin E fat emulsions tolerate 3 cycles and the particle size is about 220nm.
Analogs including Dihydrocucurbitacin B will have the similar results as made in the similar process.

**Example 16 the freeze-thaw resistant clevidipine butyrate fat emulsion injections**

The formula of the fat emulsions is based on the formula of Cleviprex (clevidipine butyrate emulsion, 0.5mg/ml) (20% soybean oil, 1.2%EPC, 0.03%oleic acid, 2.25% glycerol and 0.005% EDTA-2Na), shown in Table 67.

**Table 67 formula of clevidipine butyrate injections of different glycerolconcentrations**

| Ingredients | Glycerol (w/v) | | | | |
|---|---|---|---|---|---|
| | 2.25% | 10.0% | 15.0% | 20.0% | 40.0% |
| ClevidipineButyrate | 0.5g | 0.5g | 0.5g | 0.5g | 0.5g |
| EPC | 12g | 12g | 12 g | 12g | 12 g |
| LCT (Soybean Oil) | 200 g | 200 g | 200 g | 200 g | 200 g |
| Oleic Acid | 0.3g | 0.3g | 0.3g | 0.3g | 0.3g |
| Glycerol | 22.5g | 100.0g | 150.0 g | 200.0 g | 400.0 g |

(continued)

| Ingredients | Glycerol (w/v) | | | | |
|---|---|---|---|---|---|
| | 2.25% | 10.0% | 15.0% | 20.0% | 40.0% |
| EDTA-2Na | 0.05g | 0.05g | 0.05g | 0.05g | 0.05g |
| Sodium Hydroxide | proper | proper | proper | proper | proper |
| Injection Water up to | 1000mL | 1000 mL | 1000 mL | 1000 mL | 1000mL |

Process: (1) the making of the water phase: blend the glycerol and the EDTA-2Na with injection water, and heat it to 60°C for use; (2) the making of the oil phase: heat the soybean oil to 60°C, blend the clevidipine butyrate, EPC and oleic acid in it when stirring for use; (3) the making of the pre-emulsion: add the solution (2) to the solution (1) for 10000rpm shear, 20min to obtain the pre-emulsion; (4) Homogenization: the solution is homogenized twice in a microfluider under the pressure 20000psi and regulated to PH 8.0; (5) Filtration and Bottling: the emulsion obtained is filtered by 0.8um microfilter, bottled and sealed; (6) Sterilization: the clevidipine butyrate injections are obtained after sterilized in 121±1°C for 10 min.

HPLC methodostade-cylsilane, ODS column; 0.05mol/L sodium dihydrogen phosphate (pH4.0 by dilute phosphoric acid)-acetonitrile- methanol (50:30:20) as mobile phase; the wavelength 220nm) is used to detect the related substances and calculated by area normalization as shown in Table 68.

**Table 68 related substances/degradation products of different clevidipinebutyrate injections of different glycerol concentrations**

| | Glycerol (w/v) | | | | |
|---|---|---|---|---|---|
| | 2.25% | 10.0% | 15.0% | 20.0% | 40.0% |
| Related Substances/Degradation Products | 0.31% | 0.22% | 0.13% | 0.16% | 0.21% |

The table suggests related substances/degradation products of the clevidipine butyrate injection will fall as the glycerol increases, however when the glycerolconcentration reaches 40%, it goes up instead. Meanwhile the inventors find the emulsions of the said formula (40%) are of high viscosity, and the injection tends to aggregate and is more difficult to filter.

Although the inventors have made a detailed description and list of the invention, it is understood that for those skilled in the art, any modification, and/or variation, or equivalent substitution without departing from the spirit of the invention, and the terms for describing and understanding the invention, should not be for the use in limiting the invention.

**Claims**

1. A use of high-concentrationglycerol in freeze-thaw resistant fat emulsions, wherein the concentration of thehigh-concentrationglycerol in the emulsions is greater than or equal to 3 w/v %.

2. The use of claim 1, wherein the maximum concentrationof the high-concentrationglycerol in the emulsions is 50 w/v%.

3. The use of claim 1, wherein the glycerol is greater than or equal to the 1/3 amount of the oil in the freeze-thaw resistant fat emulsions where the concentration of the oil is 2%-30 w/v %.

4. The use of claims 1-3, wherein the concentration of the glycerol in the emulsions is 5-40%, preferably 7.5%-30%, more preferably 7.5%-15%.

5. A freeze-thaw resistant fat emulsion mainly comprising oil, glycerol, phospholipids and water, wherein the concentration of the glycerol in the emulsion is greater than or equal to 3 w/v %.

6. The freeze-thaw resistant fat emulsion of claim 5, wherein the maximum concentration of the glycerol in the emulsion is 50 w/v %.

7. The freeze-thaw resistant fat emulsion of claim 5, wherein the glycerolis greater than or equal to 1/3 amount of the oil in the emulsion where the concentration of the oil in the emulsion is 2 w/v %-30 w/v %.

8. The freeze-thaw resistant fat emulsion of claims 5-7, wherein the freeze-thaw resistant fat emulsion comprises pH regulator, and the concentration of the pH regulator enables the emulsion to be at pH 4.5-10.1

9. The freeze-thaw resistant fat emulsion of claims 5-7, wherein the emulsion contains drugs selecting from dexamethasone palmitate, CoQ10, propofol, anisole, asarone, elemene, curcumin, tanshinone IIA, prostaglandin E1, limaprost, ketoprofen isopropyl ester, malotilate, vitamin K1, cucurbitacin E, dihydrocucurbitacin B, cleviprex.

10. The freeze-thaw resistant fat emulsion of claim 9, wherein the concentration of the glycerol in the emulsion is greater than or equal to 4.5%, preferably 5%-40%, more preferably 5%-30%, most preferably 5%-20%.


**Amended claims under Art. 19.1 PCT**

1. A use of high-concentration glycerol in freeze-thaw resistant fat emulsions, wherein the concentration of the high-concentration glycerol in the emulsions is greater than or equal to 3 w/v %; the fat emulsion is a fat emulsion of 1.2% or 1.8% phospholipids.

2. The use of claim 1, wherein the maximum concentration of the high-concentration glycerol in the emulsions is 50 w/v%.

3. The use of claim 1, wherein the glycerol is greater than or equal to the 1/3 amount of the oil in the freeze-thaw resistant fat emulsions where the concentration of the oil is 2%-30 w/v %.

4. The use of claims 1-3, wherein the concentration of the glycerol in the emulsions is 5-40%, preferably 7.5%-30%, more preferably 7.5%-15%; the freeze-thaw resistant fat emulsions tolerate at least three times freeze-thaw experiments.

5. A freeze-thaw resistant fat emulsion mainly comprising oil, glycerol, 1.2% or 1.8% phospholipids and water, wherein the concentration of the glycerol in the emulsion is greater than or equal to 3 w/v %.

6. The freeze-thaw resistant fat emulsion of claim 5, wherein the maximum concentration of the glycerol in the emulsion is 50 w/v %.

7. The freeze-thaw resistant fat emulsion of claim 5, wherein the glycerol is greater than or equal to 1/3 amount of the oil in the emulsion where the concentration of the oil in the emulsion is 2 w/v %-30 w/v %.

8. The freeze-thaw resistant fat emulsion of claims 5-7, wherein the freeze-thaw resistant fat emulsion comprises pH regulator, and the concentration of the pH regulator enables the emulsion to be at pH 4.5-10.1

9. The freeze-thaw resistant fat emulsion of claims 5-7, wherein the emulsion contains drugs selecting from dexamethasone palmitate, CoQ10, propofol, anisole, asarone, elemene, curcumin, tanshinone IIA, prostaglandin E1, limaprost, ketoprofen isopropyl ester, malotilate, vitamin K1, cucurbitacin E, dihydrocucurbitacin B, cleviprex.

10. The freeze-thaw resistant fat emulsion of claim 9, wherein the concentration of the glycerol in the emulsion is greater than or equal to 4.5%, preferably 5%-40%, more preferably 5%-30%, most preferably 5%-20%.

11. The freeze-thaw resistant fat emulsion of claim 5, wherein the concentration of the glycerol is 5%-50%; the freeze-thaw resistant fat emulsion tolerates at least three times freeze-thaw experiments.

12. The freeze-thaw resistant fat emulsion of claim 11, wherein the concentration of the glycerol is 7.5%-15%; the freeze-thaw resistant fat emulsion tolerates at least three times freeze-thaw experiments.

# INTERNATIONAL SEARCH REPORT

| International application No. |
| --- |
| PCT/CN2015/080882 |

**A. CLASSIFICATION OF SUBJECT MATTER**

A61K 9/107 (2006.01) i; A61K 47/10 (2006.01) i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

DATABASE: WPI, EPODOC, CNPAT, CNABS, CPRSABS, MOABS, HKABS, TWABS, DWPI, SIPOABS, CPEA, JPABS, CNTXT, EPTXT, USTXT, WOTXT, CNKI, CA, EMBASE, MEDLINE

KEYWORDS: glycerin, glyceroloil, phospholipid, fat, emulsion, oil

**C. DOCUMENTS CONSIDERED TO BE RELEVANT**

| Category* | Citation of document, with indication, where appropriate, of the relevant passages | Relevant to claim No. |
| --- | --- | --- |
| X | CN 102805728 A (NANJING ZHENGDA TIANQING PHARM CO LTD) 05 December 2012 (05.12.2012) , see claim 1 | 1-10 |
| X | CN 101199480 A (XIAN LIBANG MEDICINE SCI & TECHNOLOGY CO) 18 June 2008 (18.06.2008) , see claim 1, example 1 | 1-10 |

☐ Further documents are listed in the continuation of Box C.  ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| Date of the actual completion of the international search | Date of mailing of the international search report |
| --- | --- |
| 31 August 2015 | 10 September 2015 |

| Name and mailing address of the ISA State Intellectual Property Office of the P. R. China No. 6, Xitucheng Road, Jimenqiao Haidian District, Beijing 100088, China Facsimile No. (86-10) 62019451 | Authorized officer LI, Fengyun Telephone No. (86-10) 62089164 |
| --- | --- |

Form PCT/ISA /210 (second sheet) (July 2009)

**INTERNATIONAL SEARCH REPORT**
Information on patent family members

International application No.

PCT/CN2015/080882

| Patent Documents referred in the Report | Publication Date | Patent Family | Publication Date |
|---|---|---|---|
| CN 102805728 A | 05 December 2012 | CN 102805728 B | 04 December 2013 |
| CN 101199480 A | 18 June 2008 | CN 101199480 B | 28 July 2010 |

Form PCT/ISA /210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201210455782

### Non-patent literature cited in the description

- **COLLOIDS ; SURFACES B.** *Biointerfaces,* 2004, vol. 37, 43-47
- **HAUMONT D et al.** Effect of liposomal content of lipid emulsions on plasma lipid concentrations in low birth weight infants receiving parenteral nutrition. *J Pediatr.,* November 1992, vol. 121 (5), 759-63
- Lipid emulsions as a novel system to reduce the hemolytic activity of lytic, agents: mechanism of the protective effect. *European Journal of Pharmaceutical Sciences,* 2000, vol. 9, 285-290
- *J Child Neurol,* 2000, vol. 15, 702-704
- *Brain and Development,* 2007, vol. 29 (7), 421-424